# EUROPEAN PATENT APPLICATION

(11) **EP 4 806 994 A1**
(43) Date of publication of application: **16.09.2026**
(21) Application number: 25382229.0
(22) Date of filing: 12.03.2025
(51) Int. Cl.: C12N 9/18, A21D 8/04, C11D 3/386, C12N 9/20

(54) **SYNTHETIC LIPASE ENZYMES AND USES THEREOF**

(71) Applicant: Asociación Centro de Investigación Cooperativa en Biociencias - CIC bioGUNE, 48160 Derio (ES)
(72) Inventor: JIMÉNEZ OSÉS, Gonzalo, E-48160 Derio, Vizcaya (ES); PECCATI, Francesca, E-48160 Derio, Vizcaya (ES); SEGOVIA MARTÍN, Cristina María, E-48160 Derio, Vizcaya (ES)
(74) Representative: ABG Intellectual Property Law, S.L.

(57) **Abstract**

The present invention relates to lipase A enzyme variants, methods of production of the same and uses thereof.

## Description

### FIELD OF INVENTION

The present invention relates to a modified enzyme, in particular lipase A, methods to obtain the modified enzyme and uses of the enzyme.

### BACKGROUND OF THE INVENTION

The stability of proteins, including thermostability, oxidation stability, tolerance to extreme pH conditions or to the presence of organic cosolvents, is a determinant of their utility in industrial and biomedical applications, as it underpins their resilience under extreme processing conditions. Over the past decades, diverse strategies have been developed to enhance protein stability and functionality. Directed evolution (DE) methodologies and more particularly error-prone polymerase chain reaction (epPCR), DNA shuffling, and iterative saturation mutagenesis (ISM), have proven effective by leveraging random mutagenesis followed by high-throughput screening. While these methods have afforded robust proteins, they often involve generating large libraries of mutant proteins, which must then be experimentally screened to identify those with the desired properties. To overcome these challenges, computational tools such as Rosetta or FoldX and machine-learning algorithms are being integrated into the protein engineering pipeline to predict mutations likely to be beneficial, dramatically reducing library size and focusing experimental variants with high probability of enhancing the desired properties. More recently, advanced deep-learning software tools such as RoseTTAFold and AlphaFold have transformed protein engineering by providing highly accurate structure predictions, allowing researchers to identify key residues for mutagenesis, thereby reducing experimental effort.

*Bacillus subtilis* lipase A (LipA) is an extensively studied mesophilic enzyme renowned for its industrial relevance and alkaline pH tolerance. Despite its utility, lipase A native thermostability and catalytic efficiency limits its applicability under stringent conditions. As such the present work focuses on improving lipase A thermostability and catalytic efficiency.

### SUMMARY OF THE INVENTION

The inventors of the present invention have identified mutations in the lipase A wild-type sequence predicted to stabilize the enzymes folded state, resulting in variants with higher unfolding temperatures values (Tₘ) that correlate well with the changes in folding free energy (ΔΔG_{f}) as calculated by a computational method named mAFmin by Peccati, F. et al. (J. Chem. Inf. Model. 2023, 63, 898-909).

A first aspect of the invention relates to a lipase A enzyme variant having at least 80% identity with an amino acid sequence according to SEQ ID NO: 1, wherein the enzyme variant is characterized by comprising the mutations Y49E, D144E/S, Y161T, and S167D, and wherein the enzyme variant has improved thermostability and/or catalytic efficiency in comparison with the enzyme according to SEQ ID NO: 1.

A second aspect relates to a lipase A enzyme variant having at least 80% identity with an amino acid sequence according to SEQ ID NO: 1, from here onwards the enzyme II of the invention, wherein the enzyme variant is characterized by comprising the mutations Y49E, N50K, and R57K, and wherein the enzyme variant has improved thermostability and/or catalytic efficiency in comparison with the enzyme according to SEQ ID NO: 1.

Another aspect of the present invention relates to a polynucleotide encoding the lipase A enzyme variant according to the invention.

A further aspect of the invention relates to a vector comprising the polynucleotide according to the invention.

One more aspect of the invention relates to a host cell comprising the polynucleotide according to the invention or the vector according to the invention.

Another aspect of the invention relates to a method for obtaining the lipase A enzyme variant according to the invention comprising the steps of:
a) culturing a host cell according to the invention;
b) recovering the lipase A enzyme variant from the culture medium or from the cell.

Yet another aspect refers to the use of the lipase A enzyme variant according to the invention as an industrial biocatalyst.

One more aspect refers to the use of the lipase A enzyme variant according to the invention in food processing.

Another aspect relates to the use of the lipase A enzyme variant according to the invention as a detergent additive.

A final aspect relates to the use of the lipase A enzyme variant according to the invention in chemical synthesis of pharmaceutical, agrochemical and/or pesticide compounds.

### BRIEF DESCRIPTION OF THE FIGURES

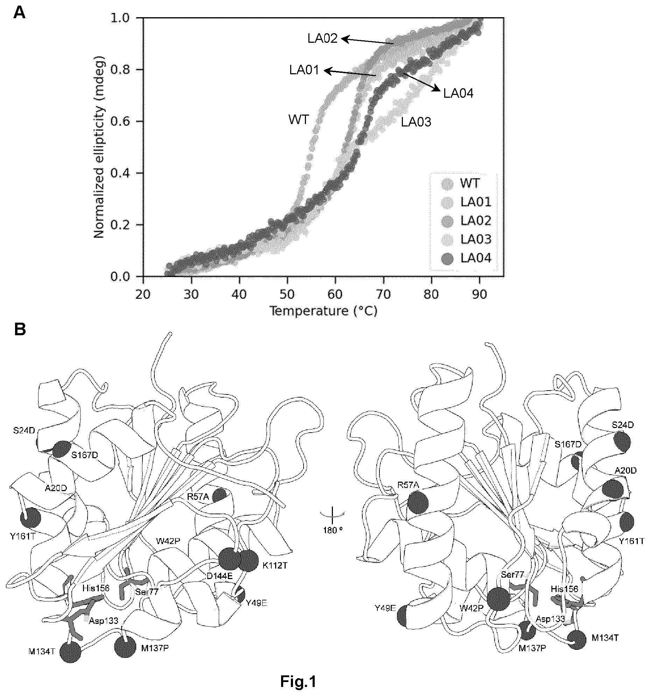

**Figure 1A)**Thermal unfolding of lipase A variants in comparison to wild-type (WT) measured by circular dichroism (CD) spectroscopy. **B)** Crystallographic structure of wild-type lipase A indicating relative positions of mutations in variant LA04 as spheres and catalytic residues as sticks. Two views of the structure rotated by 180° are presented.

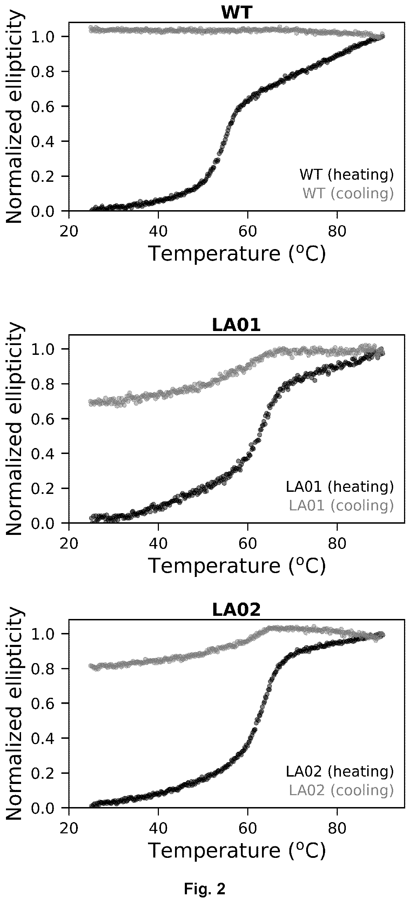

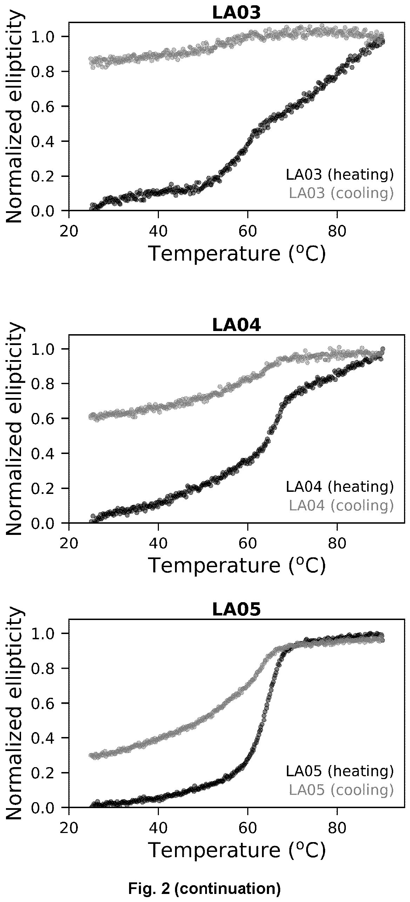

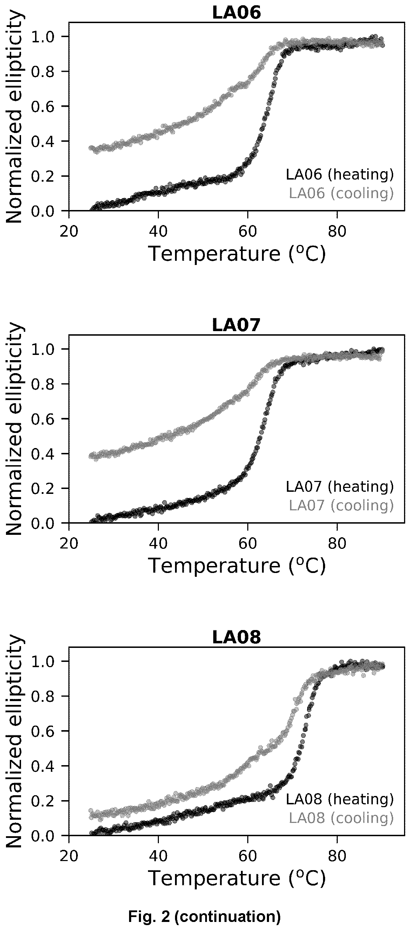

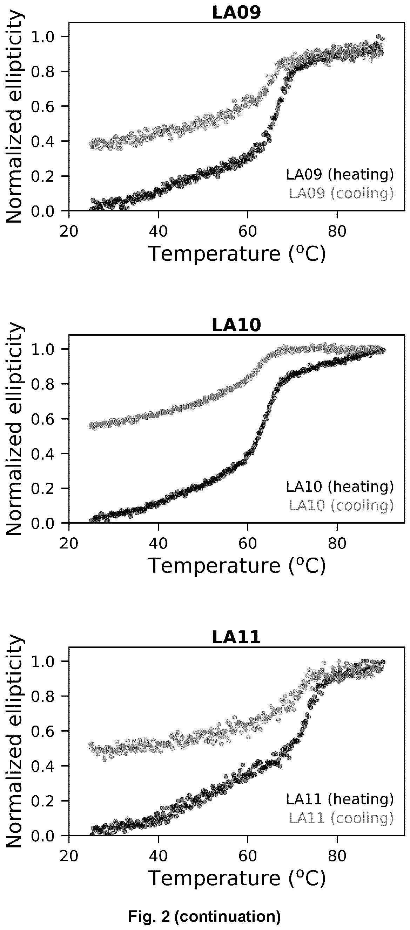

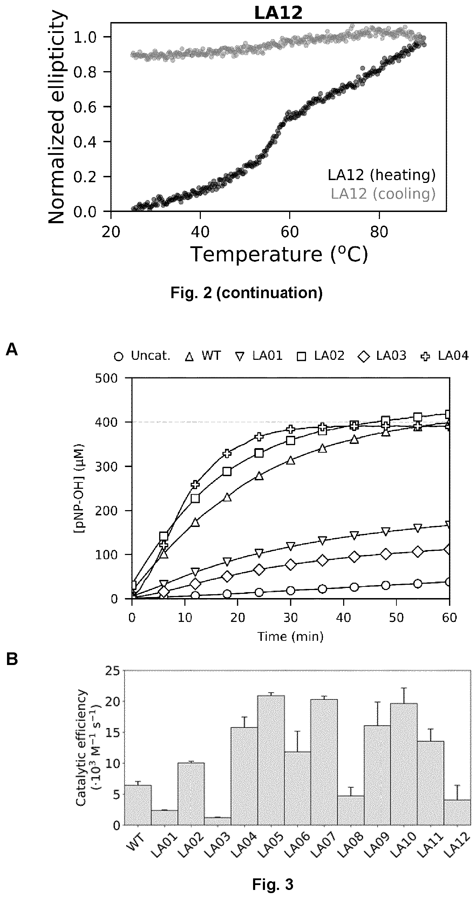

**Figure 2****.** Thermal denaturation and refolding profile of wild-type lipase A and its engineered variants monitored by circular dichroism (CD) spectroscopy during the heating (black) and cooling (grey) phases.

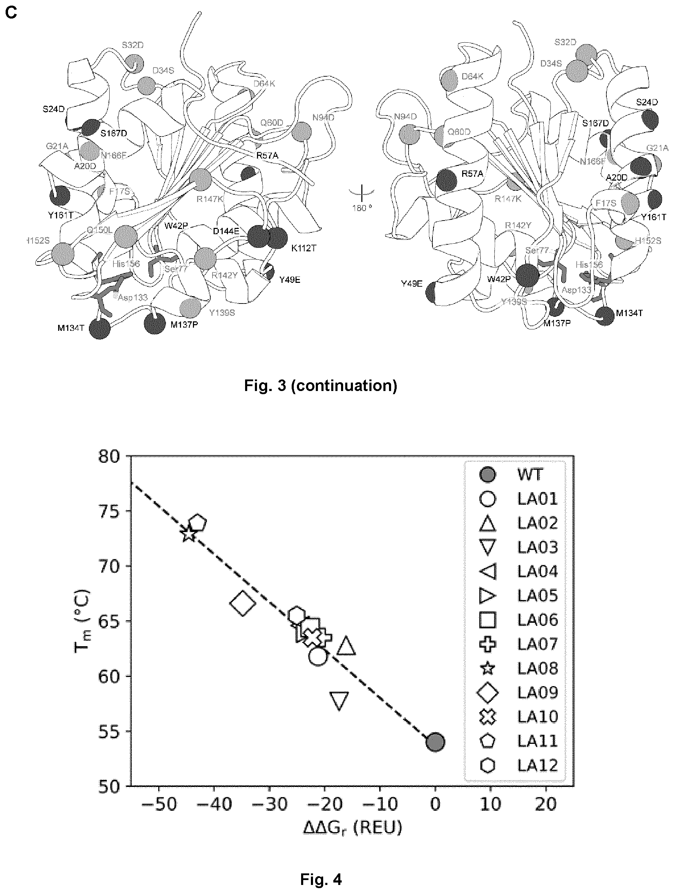

**Figure 3A)**Representative plot from monitoring of the hydrolysis of *para-*nitrophenylbutyrate (Bu-pNP) (400 µM) in the absence (Uncat.) or presence of enzyme (100 nM) in Tris buffer (20 mM, 120 mM NaCl, pH 8.0) and 5% acetonitrile at 25 °C by UV-VIS (405 nm). **B)** Catalytic efficiencies (k_{cat}/K_{M}) determined by Lineweaver-Burk plots derived from data obtained by reacting 100 nM of enzyme with increasing amounts of Bu-pNP (100 to 1000 µM) in Tris buffer (20 mM, 120 mM NaCl, pH 8.0) and 5% acetonitrile at 25 °C by UV-VIS (absorbance measured at λ=405 nm). Standard deviation of these measurements (n=3) is represented with vertical capped lines. C) Crystallographic structure of wild-type lipase A indicating relative positions of mutations in variant LA11 as spheres and catalytic residues as sticks. Two views of the structure rotated by 180° are presented.
**Figure 4****.** Representation of experimental Tₘ vs mAFmin ΔΔG_{f} for wild-type lipase A and its variants (LA01-LA12). Dashed line represents the linear regression.

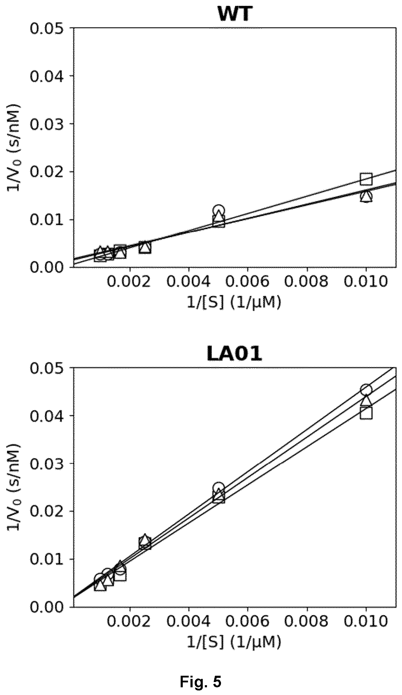

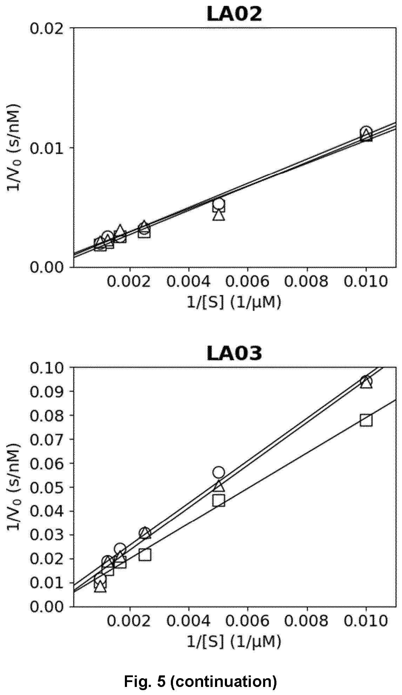

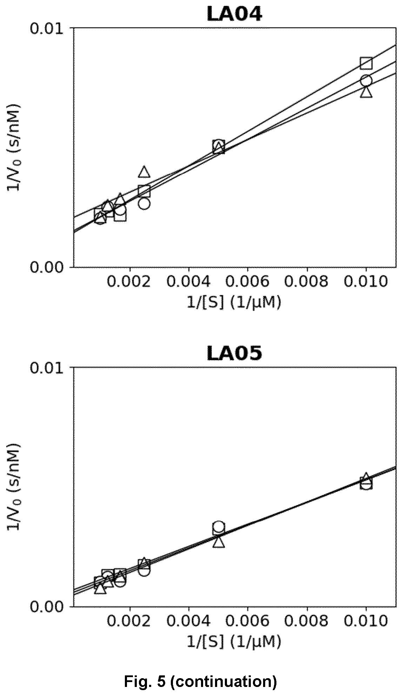

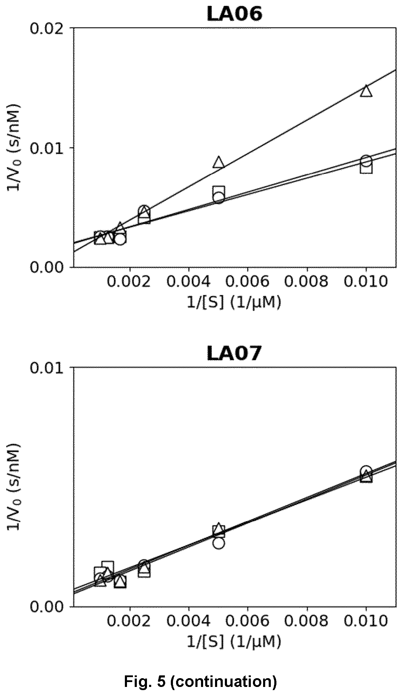

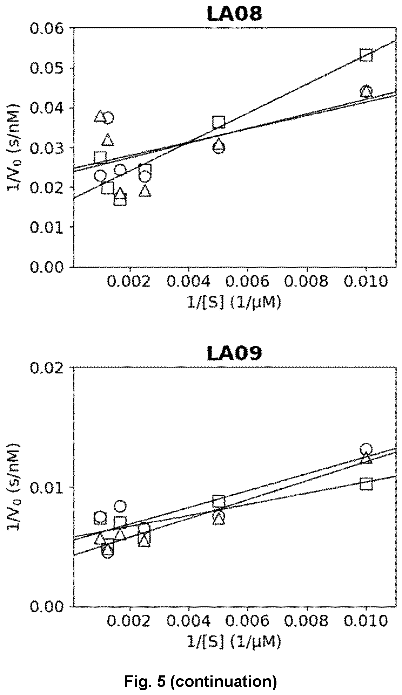

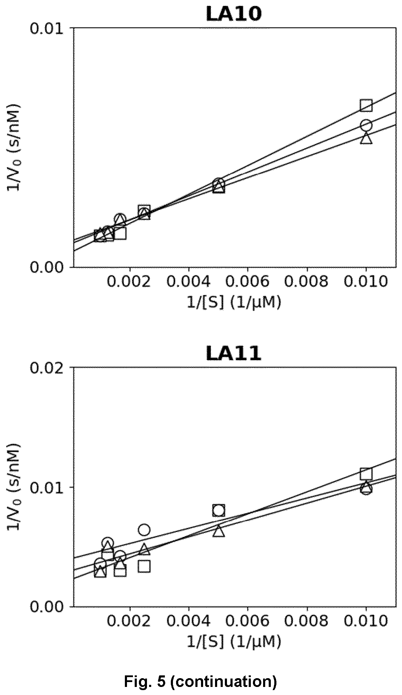

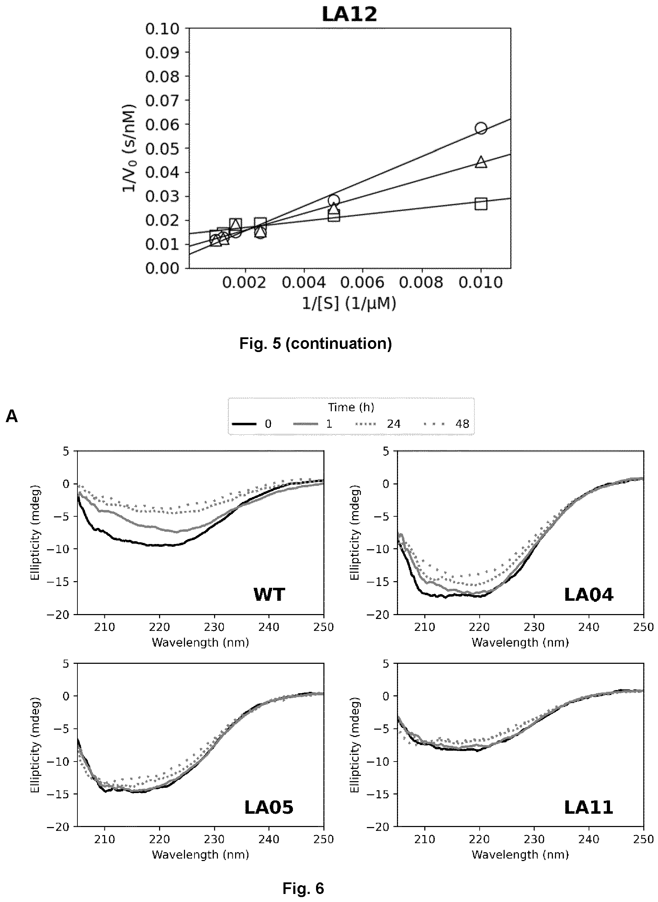

**Figure 5****.** Lineweaver-Burk plots (3 independent replicas, each represented with a different marker - circle, triangle, square) for the determination of K_{M}, k_{cat} and k_{cat}/K_{M} values of wild-type lipase A and the engineered variants using Bu-pNP as the substrate in Tris buffer (20 mM, 120 mM NaCl, pH 8.0) containing 120 mM NaCl and 5% acetonitrile at 25 °C.

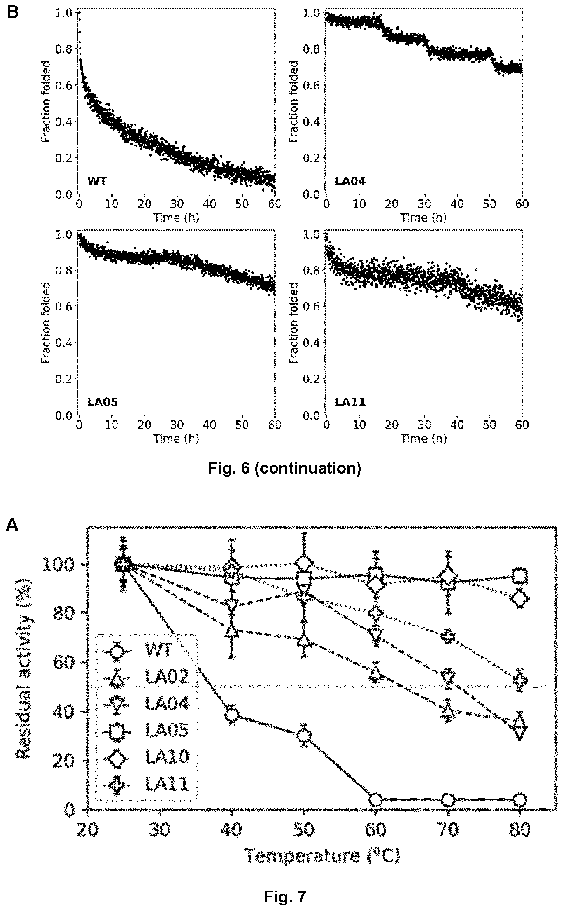

**Figure 6****.** Unfolding kinetics of lipase A variants. **A)** Monitoring of the CD spectra of wild-type lipase A and the engineered variants upon incubation at 25 °C for 60 h. **B)** Fraction of folded protein as a function of time calculated from the ellipticity at 222 nm.
**Figure 7A)**Thermal inactivation profiles of wild-type lipase A and the designed variants. Enzymes are pre-incubated at different temperatures for 20 min in Tris buffer (20 mM, 120 mM NaCl, pH 8.0) for 20 min. Residual activity was measured as the relative conversion after 20 min reaction of the enzyme (100 nM) with Bu-pNP (250 µM) in the same buffer with 5% acetonitrile at 25 °C, where 100% is the conversion after 20 min reaction upon pre-incubation of the enzyme at 25 °C. Standard deviation of these measurements (n=3) is represented with vertical capped lines. **B)** Initial velocities for the reactions of the enzymes (20 nM) with Oct-pNP (100 µM) in Tris buffer (20 mM, 120 mM NaCl, pH 8.0) and increasing amounts of DMSO at 25 °C. Standard deviation of these measurements (n=3) is represented with vertical capped lines.

### DETAILED DESCRIPTION OF THE INVENTION

The inventors of the present invention have identified mutations in the *Bacillus subtilis* lipase A wild-type sequence predicted to stabilize the enzyme folded state, resulting in variants with higher Tₘ values that correlate well with the computed changes in folding free energy. These designed enzymes exhibit slower unfolding kinetics than wild-type lipase A and partial reversibility after thermal treatment, a property not observed in the wild-type lipase A. Remarkably, many variants also display enhanced catalytic activity.

All particular embodiments and definitions provided in relation to any one aspect are equally applicable to the remaining aspects herein presented.

### Lipase A enzyme variants

A first aspect of the invention relates to a lipase A enzyme variant having at least 80% identity with an amino acid sequence according to SEQ ID NO: 1, from here onwards the enzyme of the invention, wherein the enzyme variant is characterized by comprising the mutations Y49E, D144E/S, Y161T, and S167D, and wherein the enzyme variant has improved thermostability and/or catalytic efficiency in comparison with the enzyme according to SEQ ID NO: 1.

A second aspect relates to a lipase A enzyme variant having at least 80% identity with an amino acid sequence according to SEQ ID NO: 1, from here onwards the enzyme II of the invention, wherein the enzyme variant is characterized by comprising the mutations Y49E, N50K, and R57K, and wherein the enzyme variant has improved thermostability and/or catalytic efficiency in comparison with the enzyme according to SEQ ID NO: 1.

The terms "lipase", "lipase enzyme", "lipolytic enzyme", "lipid esterase", "lipolytic polypeptide", and "lipolytic protein" or "lipase enzyme" are used interchangeably herein and refer to an enzyme in class EC 3.1.1 as defined by Enzyme Nomenclature. In particular it refers to an enzyme having lipase activity (triacylglycerol lipase, EC 3.1.1.3), acylglycerol lipase activity (EC 3.1.1.23), carboxylesterase activity (EC 3.1.1.1), cutinase activity (EC 3.1.1.74), sterol esterase activity (EC 3.1.1.13) and/or wax-ester hydrolase activity (EC 3.1.1.50). Lipase refers to a class of enzymes that catalyzes the hydrolysis of fats. Lipases are serine hydrolases, i.e. they function by transesterification generating an acyl serine intermediate. Most lipases act at a specific position on the glycerol backbone of a lipid substrate (A1, A2 or A3). A diverse array of genetically distinct lipase enzymes are found in nature, and they represent several types of protein folds and catalytic mechanisms. However, most are built on an alpha/beta hydrolase fold and employ a chymotrypsin-like hydrolysis mechanism using a catalytic triad consisting of a serine nucleophile, a histidine base, and an acid residue, usually aspartic acid. The lipase enzyme variant according to the invention is based on *Bacillus subtilis* Lipase A (LipA) according to SEQ ID NO: 1 as first disclosed in Dartois, V. et al. (Biochim. Biophys. Acta - Gene Struct. Expr. 1992, 1131, 253-260).

The expression "lipase A enzyme variant" as used herein refers to those enzymes derived from the lipase A enzyme having an amino acid sequence according to SEQ ID NO: 1 by means of modification, insertion and/or deletion of one or more amino acids, provided that the function of the lipase A enzyme remains the same or is improved in terms of the thermostability of the enzyme and/or catalytic efficiency of the enzyme. The lipase A enzyme variant of the invention is characterized by comprising at least the mutations Y49E, D144E/S, Y161T, and S167D.

As used herein, the term "parent enzyme" or "parent lipase A" is used interchangeably with the expression "lipase A enzyme having an amino acid sequence according to SEQ ID NO: 1".

The term "improved property" as used herein in reference to a lipase A enzyme variant, refers to a characteristic associated with a lipase variant that is improved compared to the parent lipase, e.g. the lipase A enzyme having the amino acid sequence according to SEQ ID NO: 1, or compared to a lipase having the identical amino acid sequence of said variant but not having the alteration at one or more of said specified positions. Improved properties include, but are not limited to, wash performance, lipase activity, esterase activity, malodor reduction, thermal activity profile, thermostability, pH activity profile, pH stability, tolerance to organic cosolvents, substrate specificity, improved surface properties, product specificity, increased stability including stability during the main wash process, improved stability under storage conditions, and chemical stability. In terms of the present invention the lipase A enzyme variant has the properties of thermostability and/or catalytic efficiency improved when compared with the lipase A enzyme having the amino acid sequence according to SEQ ID NO: 1.

The term "thermostability" or "thermal stability" as used herein refers to the ability of the enzyme to resist irreversible inactivation (usually by denaturation) at a relatively high temperature. The term "improved thermostability" in the present context refers to the capacity of the enzyme variant to have a higher retention of lipase activity after a period of incubation at a temperature than the parent enzyme and/or having a higher melting or unfolding temperature (Tₘ).

Thermostability of lipase A enzymes can be measured in several ways such as, without limitation, incubating enzyme samples without substrate for a defined period of time (e.g. 10 min or 1 to 30 min) at an elevated temperature compared to the temperature at which the enzyme is stable for a longer time (days). Following the incubation at an elevated temperature the enzyme sample is assayed for residual activity at the permissive temperature of e.g. 30 °C. (alternatively 25-50 °C or even up to 70 °C). Residual activity is calculated as relative to a sample of the enzyme that has not been incubated at the elevated temperature. The resulting thermal denaturation profile (temperature versus residual activity) can be used to calculate the temperature at which 50% residual activity is obtained. This value is defined as the half-life temperature or thermal midpoint of inactivation value (T₅₀).

Thermostability can also be measured as enzyme inactivation as a function of time. Here enzyme samples are incubated without substrate at a defined elevated temperature (e.g. 76 °C) for various time periods (e.g. between 10 sec and 30 min) and following incubation assayed for residual activity at the permissive temperature of e.g. 30 °C. (alternatively 25-70 °C. or even higher). Residual activity at each temperature is calculated as relative to an enzyme sample that has not been incubated at the elevated temperature. The resulting inactivation profile (time versus residual activity) can be used to calculate the time at which 50% residual activity is obtained. This is usually given as T₅₀.

Thermostability can also be predicted through computational protocols which combine the three-dimensional (3D) structure of the enzyme variant and energy minimization, with thermostability variation calculated as the difference between the folding free energy of the enzyme and that of the parent enzyme (ΔΔG_{f}), as explained in detail in the Examples of the present specification and in Peccati, F. et al. (J. Chem. Inf. Model. 2023, 63, 898-909).

Another method to determine the thermostability of an enzyme is thermal unfolding, wherein the loss of 3D structure is measured in an enzyme sample as the temperature increases. When the temperature rises, the increased kinetic energy can disrupt the non-covalent interactions (like hydrogen bonds, ionic bonds, and hydrophobic interactions) that hold the protein in its folded state. This causes the protein to unfold or denature, losing its functional shape. Thermal unfolding can be determined by several methods including, without limitation, differential scanning calorimetry (DSC), fluorescence spectroscopy, circular dichroism (CD) spectroscopy, infrared (IR) spectroscopy, and mass spectrometry (MS). This can be used to calculate the Tₘ (melting or unfolding temperature), which is the temperature at which 50% of a protein or enzyme population undergoes thermal denaturation. This method is further detailed in the Examples section of the present specification.

In a preferred embodiment, the enzyme of the invention has a thermostability which is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% or more improved in comparison with the lipase A enzyme having an amino acid sequence according to SEQ ID NO: 1, as measured by any of the methods herein described.

In a preferred embodiment of the enzyme of the invention, the improvement in thermostability is of at least 1 °C in the unfolding temperature of the enzyme variant, preferably of at least 2 °C, at least 3 °C, at least 4 °C, at least 5 °C, at least 6 °C, at least 7 °C, at least 8 °C, at least 9 °C, at least 10 °C, at least 11 °C, at least 12 °C, at least 13 °C, at least 14 °C, at least 15 °C, at least 16 °C, at least 17 °C, at least 18 °C, at least 19 °C, at least 20 °C, at least 21 °C, at least 22 °C, at least 23 °C, at least 24 °C, at least 25 °C or more in the unfolding temperature of the enzyme variant.

In another preferred embodiment of the enzyme of the invention, the unfolding temperature is measured by circular dichroism spectroscopy at 222 nanometers (nm).

The term "catalytic efficiency" as used herein refers to the specificity of an enzyme for a substrate and is defined as the k_{cat}/K_{M} of an enzyme. The terms "k_{cat}" and "K_{M}" are known to those skilled in the art and are described in Enzyme Structure and Mechanism, 2nd ed. (Fersht; W. H. Freeman: NY, 1985; pp 98-120). The term "k_{cat}", often called the "turnover number", is defined as the maximum number of substrate molecules converted to products per active site per unit time, or the number of times the enzyme turns over per unit time. k_{cat}=Vₘₐₓ/[E], where [E] is the enzyme concentration and Vₘₐₓ is the maximum reaction rate for a given [E] (Fersht, vide supra).

The catalytic efficiency of an enzyme can be determined by several methods and techniques such as, without limitation, Michaelis-Menten plot, Lineweaver-Burk plot, Eadie-Hofstee plot, direct measurement of product formation, and stopped-flow spectrophotometry.

In a preferred embodiment, the enzyme of the invention has a catalytic efficiency which is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% or more improved in comparison with the lipase A enzyme having an amino acid sequence according to SEQ ID NO: 1, as measured by any of the methods herein described.

In another preferred embodiment of the enzyme of the invention, the improvement in catalytic efficiency is of at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% or more improvement in comparison with the lipase A enzyme having an amino acid sequence according to SEQ ID NO: 1, as measured by any of the methods herein described.

In a preferred embodiment, the enzyme of the invention has a thermostability which is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% or more improved in comparison with the lipase A enzyme having an amino acid sequence according to SEQ ID NO: 1, as measured by any of the methods herein described and a catalytic efficiency which is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100% or more improved in comparison with the lipase A enzyme having an amino acid sequence according to SEQ ID NO: 1, as measured by any of the methods herein described.

In another preferred embodiment of the enzyme of the invention, the improvement in catalytic efficiency is determined spectrophotometrically at 25 °C through the monitoring of hydrolysis of esters containing 1,4-nitrophenol.

The enzyme of the invention is characterized by having at least 80% identity with an amino acid sequence according to SEQ ID NO: 1. In a preferred embodiment, the enzyme of the invention has at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, identity with the amino acid sequence according to SEQ ID NO: 1.

The terms "identity", "identical" or "percent identity" in the context of two or more amino acid or nucleotide sequences, refer to two or more sequences or subsequences that are the same or have a specified percentage of nucleotide or amino acid residues that are the same, when compared and aligned (introducing gaps, if necessary) for maximum correspondence, not considering any conservative substitutions as part of the sequence identity. The percent identity can be measured using sequence comparison software or algorithms or by visual inspection. Various algorithms and software are known in the art that can be used to obtain alignments of amino acid or nucleotide sequences, such as, the Needleman-Wunsch algorithm and the Smith-Waterman algorithm. Publicly available software programs can be used to align sequences, such as, EMBOSS Needle (https://www.ebi.ac.uk/idispatcher/psa/emboss_needle), NIH Basic Local Alignment Search Tool (BLAST) (https://blast.ncbi.nlm.nih.gov/BlastAlign.cgi), and Clustal Omega (https://www.ebi.ac.uk/jdispatcher/msa/clustalo). Appropriate parameters for maximal alignment by particular alignment software can be determined by one skilled in the art. In certain embodiments, the default parameters of the alignment software are used. In certain embodiments, the percentage identity "X" of a first nucleotide sequence to a second nucleotide sequence is calculated as 100 x (Y/Z), where Y is the number of nucleotide residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length which is substantially the same as the length of the first sequence.

For instance, 95% identical to a reference sequence according to the present invention, the parameters are set such that the percentage of identity is calculated over the full length of the reference nucleotide sequence and that gaps in homology of up to 5% of the total number of nucleotides in the reference sequence are allowed.

In a preferred embodiment of the enzyme of the invention, the enzyme variant sequence identity is calculated over the whole length of both sequences being compared. In another particular embodiment of the enzyme of the invention, the enzyme variant sequence identity is calculated using the Needleman-Wunsch algorithm.

As previously mentioned, the lipase A enzyme variant is obtained by means of modification, insertion and/or deletion of one or more amino acids, such that the thermostability and/or catalytic efficiency of the enzyme is improved. According to the first aspect of the invention the enzyme variant is characterized by comprising the mutations Y49E, D144E/S, Y161T, and S167D.

As the skilled person in the field is aware, the mutations in a protein amino acid sequence can be defined by a single letter (corresponding to an amino acid in the amino acid single letter code) which indicates the amino acid in the parent sequence, followed by a numeral which indicates the position of the mutation in the amino acid sequence, followed by another single letters (also corresponding to an amino acid in the amino acid single letter code) which indicates the amino acid replacing the original amino acid. Therefore, as an example, Y49D means that the tyrosine (Y) at position 49 in the amino acid sequence SEQ ID NO: 1 has been replaced by aspartic acid (D). In another example, D144E/S, means that the aspartic acid at position 144 has been replaced by glutamic acid (E) or by serine (S). The position of the amino acids can be numbered taking into consideration the first methionine resulting from recombinant expression in *E. coli* or not. In the present case, the positions are numbered not taking into consideration the first methionine.

In a preferred embodiment, the enzyme of the invention comprises the mutations Y49E, D144E, Y161T, and S167D according to SEQ ID NO: 2. In another preferred embodiment, the enzyme of the invention consists of the amino acid according to SEQ ID NO: 2.

In a preferred embodiment, the enzyme of the invention comprises the mutations Y49E, D144S, Y161T, and S167D according to SEQ ID NO: 3. In another preferred embodiment, the enzyme of the invention consists of the amino acid according to SEQ ID NO: 3.

The enzyme of the intention can comprise additional mutations as long as the resulting variants have improved thermostability and/or improved catalytic efficiency compared with the enzyme according to SEQ ID NO: 1 and, preferably, as long as the resulting variants have at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, identity with the amino acid sequence according to SEQ ID NO: 1.

In a particular embodiment, the enzyme of the invention further comprises conservative amino acid substitutions.

"Conservative amino acid substitutions" result from replacing one amino acid with another having similar structural and/or chemical properties. For example, the following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

Selection of such conservative amino acid substitutions is within the capabilities of one of ordinary skill in the art and is described, for example by Dordo et al., (J. Mol. Biol, 1999, 217;721-739) and Taylor et al., (J. Theor. Biol., 1986, 119:205-218).

In a preferred embodiment, the enzyme of the invention further comprises one of the following mutations sets:
a) W42P, M134T, M137P, and D144E;
b) M134T, M137P, and D144E;
c) A20D, S24D, W42P, R57A, K112T, and D144E; or
d) A20D, S24D, R57A, K112T, M137P, and D144E.

In another preferred embodiment of the enzyme of the invention,
a) the enzyme variant further comprising the mutations W42P, M134T, M137P, and D144E according to SEQ ID NO: 4;
b) the enzyme variant further comprising the mutations M134T, M137P, and D144E according to SEQ ID NO: 5;
c) the enzyme variant further comprising the mutations A20D, S24D, W42P, R57A, K112T, and D144E according to SEQ ID NO: 6; and
d) the enzyme variant further comprising the mutations A20D, S24D, R57A, K112T, M137P, and D144E according to SEQ ID NO: 7.

In another preferred embodiment, the enzyme of the invention consists of the sequence according to SEQ ID NO: 4, SEQ ID NO: 5, SEQ ID NO: 6, or SEQ ID NO: 7.

In a further embodiment of the enzyme of the invention, the enzyme variant comprising the mutations A20D, S24D, W42P, R57A, K112T, and D144E, further comprises the mutation M137P.

In another preferred embodiment, the enzyme of the invention comprises or consists of the sequence according to SEQ ID NO: 8.

In a further embodiment of the enzyme of the invention, the enzyme variant comprising the mutations A20D, S24D, R57A, K112T, M137P, and D144E, further comprises the mutation M134T.

In another preferred embodiment, the enzyme of the invention comprises or consists of the sequence according to SEQ ID NO: 9

In a further embodiment of the enzyme of the invention, the enzyme variant comprising the mutations W42P, M134T, M137P, and D144E, further comprises the mutations A20D, S24D, R57A, and K112T.

In another preferred embodiment, the enzyme of the invention comprises or consists of the sequence according to SEQ ID NO: 10.

In a further embodiment of the enzyme of the invention, the enzyme variant comprising the mutations W42P, M134T, M137P, D144E, A20D, S24D, R57A and K112T, further comprises the mutations F17S, Q60D, N94D, R147K, H152S and N166F.

In another preferred embodiment, the enzyme of the invention comprises or consists of the sequence according to SEQ ID NO: 11.

In a further embodiment of the enzyme of the invention, the enzyme variant comprising the mutations W42P, M134T, M137P, D144E, A20D, S24D, R57A, K112T, F17S, Q60D, N94D, R147K, H152S and N166F, further comprises the mutations S16A, I169V, and N174D.

In another preferred embodiment, the enzyme of the invention comprises or consists of the sequence according to SEQ ID NO: 12.

In a further embodiment of the enzyme of the invention, the enzyme variant comprising the mutations W42P, M134T, M137P, D144E, A20D, S24D, R57A, K112T, F17S, Q60D, N94D, R147K, H152S and N166F, S16A, I169V, and N174D, further comprises the mutations I12V, G21A, S32D, N50K, L114D, Y139S, Q150L, G155D, and Q164E.

In another preferred embodiment, the enzyme of the invention comprises or consists of the sequence according to SEQ ID NO: 13.

In a further embodiment of the enzyme of the invention, the enzyme variant comprising the mutations W42P, M134T, M137P, D144E, A20D, S24D, R57A, K112T, F17S, Q60D, N94D, R147K, H152S, N166F, S16A, I169V, , N174D, I12V, G21A, S32D, N50K, L114D, Y139S, Q150L, G155D, and Q164E, further comprises the mutations D24A, Q29K, R33A, D34S, T47A, D64K, K69D, R107A, G111T, S131T, R142W, E144S, and I157Y.

In another preferred embodiment, the enzyme of the invention comprises or consists of the sequence according to SEQ ID NO: 14.

In a further embodiment of the enzyme of the invention, the enzyme variant comprising the mutations W42P, M134T, M137P, D144E, A20D, S24D, R57A, K112T, F17S, Q60D, N94D, R147K, H152S and N166F, further comprises the mutations G21A, S32D, D34S, D64K, Y139S, R142Y, and Q150L.

In another preferred embodiment, the enzyme of the invention comprises or consists of the sequence according to SEQ ID NO: 15.

In another preferred embodiment of the enzyme II of the invention, the enzyme variant is characterized by one of the following mutations sets:
a) G21N, S24D, Y49E, N50K, V54Q, R57K, Y139E and Q164E;
b) A20D, G21Y, S24D, Q29K, Y49E, N50K, R57K, Q60N, D64K and Q164E;
c) A20D, G21N, S24D, Y49E, N50K, V54Q, S56E, R57K, D64K, Y139E, Q164E and S167E;
d) E2S, A20D, S32D, R33E, W42S, T47S, Y49E, N50K, V54E, S56E, R57K, K69E, N94D, H152S, Q164E and S167D;
e) A20D, G21Y, S24D, Q29K, N48Q, Y49E, N50K, V54Q, S56E, R57K, Q60N, K61S, D64K, Y139E, S163E, Q164E, N166A, S167E, L168Y and K170Y; or
f) E2S, A15S, F17D, A20D, G21N, S32D, R33E, W42S, T47S, Y49E, N50K, V54E, S56E, R57K, D64K, K69E, N94D, A97R, G111S, K112E, Y139E, R142E, D144K, H152S, Y161N, Q164E, S167D and Q178E.

In another preferred embodiment of the enzyme II of the invention,
a) the enzyme variant comprising the mutations G21N, S24D, Y49E, N50K, V54Q, R57K, Y139E and Q164E, comprises the sequence according to SEQ ID NO: 19;
b) the enzyme variant comprising the mutations A20D, G21Y, S24D, Q29K, Y49E, N50K, R57K, Q60N, D64K and Q164E comprises the sequence according to SEQ ID NO: 20;
c) the enzyme variant comprising the mutations A20D, G21N, S24D, Y49E, N50K, V54Q, S56E, R57K, D64K, Y139E, Q164E and S167E comprises the sequence according to SEQ ID NO: 21;
d) the enzyme variant comprising the mutations E2S, A20D, S32D, R33E, W42S, T47S, Y49E, N50K, V54E, S56E, R57K, K69E, N94D, H152S, Q164E and S167D comprises the sequence according to SEQ ID NO: 22;
e) the enzyme variant comprising the mutations A20D, G21Y, S24D, Q29K, N48Q, Y49E, N50K, V54Q, S56E, R57K, Q60N, K61S, D64K, Y139E, S163E, Q164E, N166A, S167E, L168Y and K170Y comprises the sequence according to SEQ ID NO: 23;
f) the enzyme variant comprising the mutations E2S, A15S, F17D, A20D, G21N, S32D, R33E, W42S, T47S, Y49E, N50K, V54E, S56E, R57K, D64K, K69E, N94D, A97R, G111S, K112E, Y139E, R142E, D144K, H152S, Y161N, Q164E, S167D and Q178E comprises the sequence according to SEQ ID NO: 24.

In another preferred embodiment, the enzyme of the invention further comprises a marker.

The term "marker" is used interchangeably with the terms "label" or "tag" and, as used herein, refers to components that can be attached to the enzyme variant and may allow the localization of the enzyme variant or may improve or facilitate certain properties of the enzyme variant, i.e. protein stability or resistance to degradation, or provide technical advantages during production, i.e., facilitate expression or purification. Examples of such markers/labels/tags are, without limitation, fluorescent agent or a fluorochrome (dye), such as fluorescein isocyanate (FIC), fluorescein isothiocyanate (FITC), 5-dimethylamine-1-natpthalenesulfonyl chloride (DANSC), tetramethylrhodamine isothiocyanate (TRITC), lissamine, rhodamine 8200 sulphonyl chloride (RB 200 SC), 5-TAMRA cadaverine, biotin, His tag, FLAG tag, Halo tag, MBP tag, HA tag, Myc tag, V5 tag, PA tag, fluorescent protein tag and the like.

In a preferred embodiment of the enzyme of the invention, the marker is a polyhistidine peptide. In another preferred embodiment of the enzyme of the invention, the polyhistidine peptide comprises between 2 to 10 histidines, preferably 2, 3, 4, 5, 6, 7, 8, or 9 histidines. In another preferred embodiment of the enzyme of the invention, the marker has an amino acid sequence according to SEQ ID NO: 16.

In a preferred embodiment of the enzyme of the invention, the marker is localized at the N-terminal of the enzyme variant or at the C-terminal of the enzyme variant.

In another preferred embodiment, the enzyme of the invention further comprises a flexible linker peptide between the marker and the lipase A enzyme variant.

The peptide linker may have any of a variety of amino acid sequences. Proteins can be joined by a spacer peptide, generally of a flexible nature, although other chemical linkages are not excluded. A linker can be a peptide of between about 6 and about 40 amino acids in length, or between about 6 and about 25 amino acids in length. These linkers can be produced by using synthetic, linker-encoding oligonucleotides to couple the proteins. Peptide linkers with a degree of flexibility can be used. The linking peptides may have virtually any amino acid sequence, bearing in mind that suitable linkers will have a sequence that results in a generally flexible peptide. The use of small amino acids, such as glycine, alanine and serine, are of use in creating a flexible peptide. The creation of such sequences is routine to those of skill in the art.

Suitable linkers can be readily selected and can be of any of a suitable of different lengths, such as from 1 amino acid (e.g., glycine) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, or 7 amino acids.

In a preferred embodiment of the enzyme of the invention, the flexible linker peptide has a sequence according to GₙS, wherein n is between 1 and 10, preferably wherein the flexible linker peptide is GS or GGGGS according to SEQ ID NO: 17.

In another preferred embodiment, the enzyme of the invention is immobilized to a support.

The term "support" as used herein refers to a carrier or a substrate to which the enzyme of the invention is bound to by a chemical or physical procedure. The immobilization can be carried out by well-known procedures in the art, making use of physical methods such as adsorption, encapsulation and entrapment, confinement, or making use of chemical methods such as covalent binding and cross-linking (see Chandra, P. et al. (Microbial Cell Factories 2020, 19, 169) for further details).

Examples of supports are, without limitation, an anion- or cation-exchange resin, a hydrophilic organic polymer, such as polyacrylate, polymethyl methacrylate, polymethyl methacrylate or cross-linked phenol formaldehyde condensate, or hydrophobic/mild hydrophobic organic polymer such as polyvinyl alcohol, polydivinyl-benzene and polystyrene, and any mixture thereof, an inorganic support such as silica, Celite, diatomaceous earth and perlite. More specifically, said support can be an anion-or cation-exchange resin, a hydrophilic organic polymer, such as polyacrylate, polymethyl methacrylate, polymethyl methacrylate or cross-linked phenol formaldehyde condensate, or hydrophobic organic polymer such as polyvinyl alcohol, polydivinylbenzene and polystyrene, and any mixture thereof and said desorbed component of the support is a monomer or oligomers of said resin/polymer.

In a further preferred embodiment, the enzyme of the invention is lyophilized. The term "lyophilized" or "freeze-dried", refers to a process where a substance is dehydrated by freezing it and then reducing the surrounding pressure to allow the frozen water in the material to sublimate directly from the solid phase to the gas phase.

### Other products

The enzyme of the invention can be obtained by the expression of polynucleotides comprising a sequence which encodes for the enzyme variants, said sequence being present in a vector and/or host cells.

As such a further aspect of the present invention relates to a polynucleotide encoding the lipase A enzyme variant according to the invention, from here onwards the polynucleotide of the invention.

The term "polynucleotide" or "nucleic acid" refers to deoxyribonucleic acids (DNA) or ribonucleic acids (RNA) and their polymers in either single or double stranded form. Unless specifically limited, the term encompasses nucleic acids containing known analogs of natural nucleotides that have binding capabilities similar to those of a reference nucleic acid and which are metabolized similarly to naturally occurring nucleotides. Unless otherwise indicated, a specific nucleic acid sequence also implies conservatively modified variants (e.g., substitutions with degenerate codons), alleles, orthologs, SNPs and complementary sequences, as well as sequences indicated in direct form. In particular, substitutions with degenerate codons can be obtained by creating sequences in which the third position of one or more selected (or all) codons is replaced by residues with mixed bases and/or deoxyinosine residues.

The polynucleotide of the invention can be present in a vector which can be used to express the enzyme of the invention in different host cells.

Therefore, another aspect of the present invention relates to a vector comprising the polynucleotide of the invention, from here onwards the vector of the invention.

The choice of the vector will depend on the host cell in which it is to be subsequently introduced. In a particular embodiment, the vector of the invention is an expression vector. Suitable vectors for the insertion of the polynucleotide sequence of the invention are vectors derived from prokaryotic expression vectors such as pUC 18, pUC 19, pGEX-6P-I, Bluescript, pET-21b(+) (GenScript Biotech) and their derivatives, mp I 8, mp I 9, pBR322, pMB9, ColEl, PCRI, RP4, phage and "shuttle" vectors such as pSA3 and pAT28, yeast expression vectors such as yeast 2 micron plasmid, integration plasmid, yeast episomalplasmid (YEp) vectors, similar and centromeric plasmids, expression vectors in insect cells such as vectors the pAC series and the pVL series, plant expression vectors such as vectors series pIBI, pEarleyGate, pAVA, pCAMBIA, pGSA, pGWB, pMDC, pMY, pORE and similar and expression vectors in eukaryotic cells rather than based on viral vectors (adenovirus, adenovirus associated viruses and retroviruses, and particularly, lentivirus) and non-viral vectors such as pSilencer 4.1-CMV (Ambion), pcDNA3, pcDNA3.1/hyg pHCMV/Zeo, pCR3.1 , pEFI/His, pIND/GS, pRc/HCMV2, pSV40/Zeo2, PTRACE- HCMV, pUB6N5-His, pVAXI, pZeoSV2, pCl, pSVL and pKSV-10, pBPV-1, pML2d and pTDTI. By way of illustration, the vector in which said nucleic acid sequence is introduced can be a plasmid which is or is not integrated in the genome of a host cell when it is introduced in said cell. Illustrative, non-limiting examples of vectors in which the nucleotide sequence of the invention or the gene construct of the invention can be inserted include a Tet-On inducible vector for expression in eukaryote cells. The vector of the invention can be obtained by conventional methods known by persons skilled in the art (Sambrook et al., 1989). In a particular embodiment of the vector of the invention, said vector is a vector useful for transforming bacterial cells.

The vector of the invention can be used to transform, transfect, or infect cells which can be transformed, transfected, or infected by said vector. In this sense, another aspect of the present invention relates to a host cell comprising the polynucleotide of the invention and/or the vector of the invention and/or expressing the enzyme of the invention, from here onwards the host cell of the invention. Said cells can be prokaryotic or eukaryotic. The vector of the invention can be used to transform eukaryotic cells such as yeast cells, *Saccharomyces cerevisiae*, or mammalian cells for example epithelial kidney 293 cells or U2OS cells, or HeLa cells or prokaryotic cells such as bacteria, *Escherichia coli*, e.g. BL21, or *Bacillus subtilis*, for example.

### Methods and uses of the invention

Another aspect of the present invention relates to a method for obtaining the enzyme of the invention, from here onwards the method of the invention, comprising the steps of:
a) culturing a host cell of the invention;
b) recovering the lipase A enzyme variant from the culture medium or from the cell.

The first step of the method refers to the process of growing the host cells of the invention, such as bacterial cells, in a suitable medium for their growth and expression of the polynucleotide of the invention in order to produce the enzyme of the invention. Materials and methods suitable for the maintenance and growth of bacterial cultures are well known in the art (Sambrook et al., 1989). In addition to this, methods and materials for inducing and improving the expression of proteins of interest are also well known in the art (Sambrook et al., 1989).

In a preferred embodiment of the method of the invention, the lipase A enzyme is expressed during step a) and is secreted to the medium. In another preferred embodiment of the method of the invention, the lipase A enzyme is expressed during step a) and is accumulated in the host cell, in which case the enzyme has to be recovered from the cell. In this particular embodiment, step a) can further comprise lysing the host cell and pouring the lipase A enzyme into the culture medium. Alternatively, the cells can be separated from the culture medium and subsequently lysed, obtaining the lipase A from said lysate.

The second step of the invention relates to a process of isolating and optionally purifying the enzyme of the invention from the culture medium or from the cell.

The term "isolate" refers to the process of extracting the enzymes from their biological source, such as cells or tissues. The goal is to separate the enzymes from other cellular components like nucleic acids, lipids, and other macromolecules. Isolation typically involves cell lysis, in the case that the enzyme is accumulated inside the host cell, and initial separation techniques like centrifugation or precipitation to obtain a crude enzyme extract.

The term "purification" refers to the process of refining the isolated enzyme to achieve a high level of purity. Purification techniques are used to separate the target protein from other proteins and contaminants in the crude extract. Common purification methods include, without limitation, chromatography (e.g., ion exchange, affinity, size exclusion), electrophoresis, and ultrafiltration.

The enzyme of the invention finds several uses in different contexts such as industrial applications, food applications, chemical applications and pharmaceutical applications.

One aspect of the present invention relates to the use of the enzyme of the invention as an industrial biocatalyst.

The term "industrial biocatalyst" as used herein refers to a catalyst, in the present case the enzyme of the invention, used to accelerate chemical reactions in industrial processes. These biocatalysts are employed in various industries, including pharmaceuticals, biofuels, food production, and textiles, due to their ability to conduct reactions efficiently and sustainably under mild conditions. Industrial biocatalysts have key features such as specificity, as they often catalyze specific reactions, reducing the occurrence of unwanted side reactions, and wide range of operating conditions allowing them to function at wide range of temperatures, which can be useful to lower energy consumption and/or reduce the need for harsh chemicals.

The enzyme of the invention, a microbial lipase, can catalyze the hydrolysis (and synthesis) of long-chain triglycerides to fatty acids, diacylglycerol, monoacylglycerol and glycerol known as carboxylesterase activity. In addition to this, it displays interesterification, esterification, aminolysis and alcoholysis activity which are useful in a wide range industries such as biodiesel, foods and drinks, leather, textile, detergents, pharmaceuticals and medicals.

Another aspect of the present invention relates to the use of the enzyme of the invention in food processing.

The term "food processing" as used herein refers to the process of manipulating or altering foodstuff in combination with the enzyme of the invention alone or with further components, in such a way that the processing results in either an ingestible product or a byproduct. Examples of food processing are the hydrolysis of milk fat to modify the fatty acid chain lengths, used for example to boost the flavor of cheese, or to speed up the ripening of cheese and lipolysis of fat, butter and cream, manufacturing of oil and fats, used in order to amend the assets of lipids in the glycerides, and manufacturing of vegetable oils, tea manufacturing by enzymatic breakdown of membrane lipids, quantitative determination of triacylglycerol, which is done usually with immobilized lipases, which is essential in the food industry specifically in fats and oils, soft drinks, drug industries, beverages, and also in medical diagnosis.

Another aspect of the present invention relates to the use of the enzyme of the invention as a detergent additive.

The term "detergent additive" as used herein refers to a compound which is added to a detergent, such as a laundry detergent. In the context of the present invention, the enzyme of the invention is added to a detergent in order to improve and support the function of the detergent, preferably a laundry detergent.

Another aspect of the present invention relates to the use of the enzyme of the invention in chemical synthesis of compounds, wherein the compound is selected from a pharmaceutical compound, an agrochemical compound or a pesticide compound.

The process of obtaining pharmaceutical compounds normally has several intermediate steps of synthesis and modification of the original compounds in order to obtain the final compound of interest. Lipases are useful in resolving racemic mixtures and to synthesize chiral building blocks. Lipases can be used for pharmaceuticals, agrochemicals and pesticides. "Pharmaceutical compound" as used herein refers to a chemical substance used in the diagnosis, treatment, or prevention of diseases. These compounds are the active ingredients in medications and can be formulated into various dosage forms such as tablets, capsules, injections, and topical applications. "Agrochemical compound", also known as an agrichemical, as used herein refers to a chemical product used in agriculture to manage ecosystems and improve crop production. This includes fertilizers, herbicides, insecticides, fungicides, and other chemicals that protect plants from pests and diseases. "Pesticide compound" in the present context refers to a substance or mixture of substances intended to prevent, destroy, repel, or mitigate any pest. Pests can include insects, weeds, fungi, rodents, and other organisms that can harm crops, livestock, or human health. Pesticides are used in agriculture, public health, and other settings to control said harmful organisms.

Another aspect of the present invention relates to the use of the enzyme of the invention in the production of a cosmetic product.

The term "cosmetic product" as used herein refers to any substance or mixture intended to be applied to the external parts of the human body for non-treatment purposes (such as the skin, hair, nails, lips, and external genital organs) or to the teeth and mucous membranes of the oral cavity. The primary purposes of cosmetic products are to clean, perfume, change the appearance, protect, keep in good condition, or correct body odors.

Another aspect of the present invention relates to the use of the enzyme of the invention as a biosensor.

As used herein, the term "biosensor" includes analytical devices that integrate biological or biologically derived sensitive elements, such as amino acids (e.g., cysteine), proteins, antibodies, nucleic acids, microorganisms, or cells. Sensitive components are integrated into or physically related to the physicochemical converter. The overall purpose of a biosensor is to generate discrete or continuous digital electronic signals that are proportional to a single analyte or a group of related analytes. In the present context the enzyme of the invention can be used, without limitation, for the quantitative determination of triacylglycerol, the quantification of which is essential in industries such as those related with fats and oils, soft drinks, drug industries, beverages, and also in medical diagnosis.

The present invention is further defined by the following aspects:
1. A lipase A enzyme variant having at least 80% identity with an amino acid sequence according to SEQ ID NO: 1, wherein the enzyme variant is characterized by comprising the mutations Y49E, D144E/S, Y161T, and S167D, and wherein the enzyme variant has improved thermostability and/or catalytic efficiency in comparison with the enzyme according to SEQ ID NO: 1.
2. The lipase A enzyme variant according to aspect 1 further comprising one of the following mutations sets:
   a) W42P, M134T, M137P, and D144E;
   b) M134T, M137P, and D144E;
   c) A20D, S24D, W42P, R57A, K112T, and D144E; or
   d) A20D, S24D, R57A, K112T, M137P, and D144E.
3. The lipase A enzyme variant according to aspect 2 wherein:
   a) the enzyme variant comprising the mutations W42P, M134T, M137P, and D144E comprises the sequence according to SEQ ID NO: 4;
   b) the enzyme variant comprising the mutations M134T, M137P, and D144E comprises the sequence according to SEQ ID NO: 5;
   c) the enzyme variant comprising the mutations A20D, S24D, W42P, R57A, K112T, and D144E comprises the sequence according to SEQ ID NO: 6; and
   d) the enzyme variant comprising the mutations A20D, S24D, R57A, K112T, M137P, and D144E comprises the sequence according to SEQ ID NO: 7.
4. The lipase A enzyme variant according to aspect 2 wherein the variant comprising the mutations A20D, S24D, W42P, R57A, K112T, and D144E, further comprises the mutation M137P.
5. The lipase A enzyme variant according to aspect 4, wherein the enzyme variant comprises the sequence according to SEQ ID NO: 8.
6. The lipase A enzyme variant according to aspect 2 wherein the enzyme variant comprising the mutations A20D, S24D, R57A, K112T, M137P, and D144E, further comprises the mutation M134T.
7. The lipase A enzyme variant according to aspect 6 wherein the enzyme variant comprises the sequence according to SEQ ID NO: 9.
8. The lipase A enzyme variant according to aspect 2 wherein the enzyme variant comprising the mutations W42P, K112T, M134T, M137P, and D144E, further comprises the mutations A20D, S24D, and R57A.
9. The lipase A enzyme variant according to aspect 8 wherein the enzyme variant comprises the sequence according to SEQ ID NO: 10.
10. The lipase A enzyme variant according to aspect 8 further comprising the mutations F17S, Q60D, N94D, R147K, H152S and N166F.
11. The lipase enzyme variant according to aspect 10 wherein the enzyme variant comprises the sequence according to SEQ ID NO: 11.
12. The lipase A enzyme variant according to aspect 10 further comprising the mutations S16A, I169V, and N174D.
13. The lipase enzyme variant according to aspect 12 wherein the enzyme variant comprises the sequence according to SEQ ID NO: 12.
14. The lipase A enzyme variant according to aspect 12 further comprising the mutations I12V, G21A, S32D, N50K, L114D, Y139S, Q150L, G155D, and Q164E.
15. The lipase enzyme variant according to aspect 14 wherein the enzyme variant comprises the sequence according to SEQ ID NO: 13.
16. The lipase A enzyme variant according to aspect 14 further comprising the mutations D24A, Q29K, R33A, D34S, T47A, D64K, K69D, R107A, G111T, S131T, R142W, E144S, and I157Y.
17. The lipase A enzyme variant according to aspect 16 wherein the enzyme variant comprises the sequence according to SEQ ID NO: 14.
18. The lipase A enzyme variant according to aspect 10 further comprising the mutations G21A, S32D, D34S, D64K, Y139S, R142Y, and Q150L.
19. The lipase A enzyme variant according to aspect 18 wherein the enzyme variant comprises the sequence according to SEQ ID NO: 15.
20. A lipase A enzyme variant having at least 80% identity with an amino acid sequence according to SEQ ID NO: 1, wherein the enzyme variant comprises the mutations Y49E, N50K, and R57K, and wherein the enzyme variant has improved thermostability and/or catalytic efficiency in comparison with the enzyme according to SEQ ID NO: 1.
21. The enzyme variant of aspect 20, comprising one of the following mutations sets:
   a) G21N, S24D, Y49E, N50K, V54Q, R57K, Y139E and Q164E;
   b) A20D, G21Y, S24D, Q29K, Y49E, N50K, R57K, Q60N, D64K and Q164E;
   c) A20D, G21N, S24D, Y49E, N50K, V54Q, S56E, R57K, D64K, Y139E, Q164E and S167E;
   d) E2S, A20D, S32D, R33E, W42S, T47S, Y49E, N50K, V54E, S56E, R57K, K69E, N94D, H152S, Q164E and S167D;
   e) A20D, G21Y, S24D, Q29K, N48Q, Y49E, N50K, V54Q, S56E, R57K, Q60N, K61S, D64K, Y139E, S163E, Q164E, N166A, S167E, L168Y and K170Y; or
   f) E2S, A15S, F17D, A20D, G21N, S32D, R33E, W42S, T47S, Y49E, N50K, V54E, S56E, R57K, D64K, K69E, N94D, A97R, G111S, K112E, Y139E, R142E, D144K, H152S, Y161N, Q164E, S167D and Q178E.
22. The enzyme variant of aspect 21, wherein:
   a) the enzyme variant comprising the mutations G21N, S24D, Y49E, N50K, V54Q, R57K, Y139E and Q164E, comprises the sequence according to SEQ ID NO: 19;
   b) the enzyme variant comprising the mutations A20D, G21Y, S24D, Q29K, Y49E, N50K, R57K, Q60N, D64K and Q164E comprises the sequence according to SEQ ID NO: 20;
   c) the enzyme variant comprising the mutations A20D, G21N, S24D, Y49E, N50K, V54Q, S56E, R57K, D64K, Y139E, Q164E and S167E comprises the sequence according to SEQ ID NO: 21;
   d) the enzyme variant comprising the mutations E2S, A20D, S32D, R33E, W42S, T47S, Y49E, N50K, V54E, S56E, R57K, K69E, N94D, H152S, Q164E and S167D comprises the sequence according to SEQ ID NO: 22;
   e) the enzyme variant comprising the mutations A20D, G21Y, S24D, Q29K, N48Q, Y49E, N50K, V54Q, S56E, R57K, Q60N, K61S, D64K, Y139E, S163E, Q164E, N166A, S167E, L168Y and K170Y comprises the sequence according to SEQ ID NO: 23;
   f) the enzyme variant comprising the mutations E2S, A15S, F17D, A20D, G21N, S32D, R33E, W42S, T47S, Y49E, N50K, V54E, S56E, R57K, D64K, K69E, N94D, A97R, G111S, K112E, Y139E, R142E, D144K, H152S, Y161N, Q164E, S167D and Q178E comprises the sequence according to SEQ ID NO: 24.
23. The lipase A enzyme variant according to any one of aspects 1 to 22 further comprising a marker.
24. The lipase A enzyme variant according to aspect 23 wherein the marker is a polyhistidine peptide.
25. The lipase A enzyme variant according to aspect 24 wherein the polyhistidine peptide comprises between 2 to 10 histidines.
26. The lipase A enzyme variant according to any one of aspects 23 to 25, further comprising a flexible linker peptide between the marker and the lipase A enzyme variant.
27. The lipase A enzyme variant according to aspect 26, wherein the flexible linker peptide has a sequence according to GₙS, wherein n is between 1 and 10, preferably wherein the flexible linker peptide is GS or GGGGS according to SEQ ID NO: 17.
28. The lipase A enzyme variant according to any one of aspects 1 to 27, the improvement in thermostability is of at least 1 °C in the unfolding temperature of the enzyme variant.
29. The lipase A enzyme variant according to aspect 28, wherein the unfolding temperature is measured by circular dichroism spectroscopy at 222 nanometers (nm).
30. The lipase A enzyme variant according to any one of aspects 1 to 29, wherein the improvement in catalytic efficiency is of at least 1% or more.
31. The lipase A enzyme variant according to aspect 30 wherein the improvement in catalytic efficiency is determined spectrophotometrically at 25 °C through the monitoring of hydrolysis of esters containing 1,4-nitrophenol.
32. The lipase A enzyme variant according to any of aspects 1 to 31 immobilized to a support.
33. The lipase A enzyme variant according to any of aspects 1 to 32 wherein the enzyme is lyophilized.
34. A polynucleotide encoding the lipase A enzyme variant according to any one of aspects 1 to 31.
35. A vector comprising the polynucleotide according to aspect 34.
36. A host cell comprising the polynucleotide according to aspect 34 or the vector according to aspect 35.
37. The host cell according to aspect 36 wherein the host cell expresses the lipase A enzyme variant according to any one of aspects 1 to 33.
38. A method for obtaining the lipase A enzyme variant according to any of aspects 1 to 33 comprising the steps of:
   a) culturing a host cell according to aspect 37;
   b) recovering the lipase A enzyme variant from the culture medium or from the cell.
39. Use of the lipase A enzyme variant according to any one of aspects 1 to 33 as an industrial biocatalyst.
40. Use of the lipase A enzyme variant according to any one of aspects 1 to 33 in food processing.
41. Use of the lipase A enzyme variant according to any one of aspects 1 to 33 as a detergent additive.
42. Use of the lipase A enzyme variant according to any one of aspects 1 to 33 in chemical synthesis of compounds, wherein the compound is selected from a pharmaceutical compound, an agrochemical compound or a pesticide compound.
43. Use of the lipase A enzyme variant according to any one of aspects 1 to 33 in the production of a cosmetic.
44. Use of the lipase A enzyme variant according to any one of aspects 1 to 33 as a biosensor.

The disclosure is detailed below by means of the following examples which are merely illustrative and by no means limiting the scope of the disclosure.

### EXAMPLES

### Materials and Methods

### 1. Sequence design

### Round 1

*3D model generation*: 100 models of wild-type lipase A were generated using AlphaFold (Jumper, J., et al., Nature 2021, 596, 583-589) (*monomer*, *model_3* parameters).

*Conservation analysis*: The *uniref90_hits.sto* multiple sequence alignment generated in the AlphaFold structure prediction of wild-type lipase A was reduced filtering out all sequences with an identity < 70% compared to wild-type lipase A. The reduced alignment was used to compute the by-position Shannon Entropy (Shannon, C. E., Bell Syst. Tech. J. 1948, 27, 379-423) according to eq. 1. ${SE}_{i} = - {\sum }_{j} {f}_{j} ln {f}_{j}$

Where *SEᵢ* is the Shannon Entropy at position *i*, *j* runs over all amino acid identities at position *i*, and *fⱼ* is the frequency of each amino acid identity in the multiple sequence alignment (MSA) at position *i*. The 18 positions with the highest *SEᵢ* were selected as the variable positions in *set*1*.* A second set of variable positions, *set*2, was generated from *set1* by removing three positions close to the catalytic triad (positions 132, 134, 137). The positions defined in each set are listed in Table 1.

**Table 1. Variable positions defined as set1 and set2.**

| *set*1 | *set*2 |
|---|---|
| 2 20 24 27 35 42 49 57 61 64 97 112 132 134 137 144 161 167 | 2 20 24 27 35 42 49 57 61 64 97 112 144 161 167 |

### ProteinMPNN sequence design:

100 AlphaFold models for wild-type lipase A were used as input to ProteinMPNN (Dauparas, J. et al., Science 2022, 378, 49-56). Two independent ProteinMPNN calculations were run for each AlphaFold model, one for each set of variable positions (*set*1 and *set*2)*.* For each calculation, a sampling temperature (*--sampling_temp*) of 0.1 and 1000 sequences (*--num_seq_per_target*) were requested, excluding cysteines (*--omit_AAs='C'*)*.* Each of the 200 blocks of 1000 ProteinMPNN solutions was analyzed individually by computing the *consensus* sequence (most frequent amino acid identity at each position) and one *minimal* sequence, which accumulates only mutations that occur in ≥ 70% of the solutions.

Analysis of all *consensus* and *minimal* sequences led to the selection of six engineered variants for experimental testing: varE and varF, both comprising the minimal number of mutations; LA01, the most frequent *minimal* sequence obtained from sampling *set1;* LA02, the second most frequent *minimal* sequence obtained from sampling *set1;* LA03, the most frequent *consensus* sequence obtained from sampling *set*2; LA04, the second most frequent *consensus* sequence obtained from sampling *set*1*.*

### Round 2

*3D model generation*: A model of LA04 was generated using AlphaFold (*monomer* parameters, *model_3* parameters). LA05, LA06, and LA07 are single and double mutants of LA04 that were generated by visual inspection of the LA04 AlphaFold model.

*Conservation analysis*: The *uniref90_hits.sto* multiple sequence alignment generated in the AlphaFold structure prediction of LA04 was reduced filtering out all sequences with an identity < 70% compared to LA04. Shannon Entropy on the reduced multiple sequence alignment was computed as described above, and two sets of variable positions, *set*3 (40 positions) and *set*4 (65 positions), were defined as those positions with Shannon Entropy ≥ 0.65 and ≥ 0.40, respectively. Position 137, which is proline in LA04, was removed from *set*3*.*

*ProteinMPNN sequence design*: The AlphaFold model for LA04 was used as input to ProteinMPNN. One ProteinMPNN calculation was run for each of *set*3 and *set*4, requesting three sampling temperatures (*--sampling_temp*) 0.1, 0.2, 0.3, and 1000 sequences per temperature (*--num_seq_per_target*), for a total of 3000 solutions per run, again excluding cysteines (*--omit_AAs='C'*)*.* The 3000 sequences from *set*3 and *set*4 were analyzed individually and five variants were selected for experimental testing: LA08, the solution with the lowest ProteinMPNN *global_score* obtained by sampling *set*4; LA09, the *minimal* sequence that accumulates only mutations present in ≥ 70% of the solutions from *set*4; LA10, the *minimal* sequence that accumulates only mutations present in ≥ 90% of the solutions from *set*4; LA11, the *minimal* sequence that accumulates only mutations present in ≥ 70% of the solutions from *set*3; LA12, the *minimal* sequence that accumulates only mutations present in ≥ 90% of the solutions from *set*3.

### 2. Prediction of relative thermostability of designed variants

The relative stability of all lipase A engineered variants with respect to the wild-type was calculated using a computational protocol previously described by us that combines AlphaFold structure ensembles and the Rosetta energy function. For each variant and the wild-type, 30 AlphaFold models were generated (*monomer*, *model_3* parameters) and each model was minimized using the Rosetta minimize application (flags: *-run:min_type lbfgs_armijo_nonmonotone, -run:min_tolerance 0.001*)*.* The ΔΔG_{f} value for each variant was calculated as the difference between the average of the 25 lowest scores of the variant and the same average of the wild-type scores (Table 2).

**Table 2. Predicted stability change (ΔΔG_{f}) of the twelve engineered lipase A variants with respect to the wild-type. Negative ΔΔG_{f} values indicate increased stability.**

| **SEQ ID NO:** | **variant** | **ΔΔG_{f} (REU)** |
|---|---|---|
| 2 | varE | -6.27 |
| 3 | varF | -3.96 |
| 4 | LA01 | -21.20 |
| 5 | LA02 | -16.14 |
| 6 | LA03 | -17.39 |
| 10 | LA04 | -24.50 |
| 8 | LA05 | -23.46 |
| 9 | LA06 | -22.63 |
| 7 | LA07 | -20.43 |
| 14 | LA08 | -44.59 |
| 13 | LA09 | -34.80 |
| 12 | LA10 | -22.22 |
| 15 | LA11 | -42.99 |
| 11 | LA12 | -25.07 |
| 24 | LAL201 | -20.23 |
| 22 | LAL202 | -16.18 |
| 21 | LAL203 | -9.52 |
| 19 | LAL204 | -12.91 |
| 23 | LAL205 | -6.28 |
| 20 | LAL206 | -9.86 |

### 3. Protein expression and purification

The recombinant plasmid pET-21b(+) (GenScript Biotech), encoding residues 1-181 of either the wild-type lipase A or any of the 12 designed proteins with a N-terminal 6xHis tag (HHHHHHGS - SEQ ID NO: 18) between the restriction sites *NdeI* and *XhoI* were transformed into *E. coli* BL21 (DE3) competent cells. Transformed cells were plated on Luria-Bertani (LB) agar plates containing ampicillin (50 µg/mL) and incubated overnight at 37 °C. A single colony from each plate was inoculated into 10 mL of LB broth (Lennox) supplemented with 10 µL of ampicillin (50 mg/mL) and incubated at 37 °C with shaking until the optical density at 600 nm (OD600) reached 0.6-0.8. This process was performed in triplicate (total culture volume: 30 mL). Protein expression was induced by adding 10 µL of isopropyl β-D-1-thiogalactopyranoside (IPTG, 0.5 M) and the cultures were incubated overnight at 25 °C. Cells were harvested by centrifugation at 4500 rpm for 20 minutes at 4 °C, and the resulting cell pellets were resuspended in 35 mL of lysis buffer (20 mM Tris pH 8.0, 120 mM NaCl, 2 mM imidazole, 1 mM protease inhibition cocktail - PIC). The cells were lysed by sonication (60% amplitude for 10 mins, in cycles of 10 seconds on and 20 seconds off). The lysates centrifuged at 25000 rpm for 20 minutes at 4 °C and 3.5 µL benzonase nuclease (Sigma-Aldrich) was added to the supernatant. After incubation for 30 min at room temperature, the soluble fraction was added onto a 1 mL Ni-NTA resin column (Merck), cleaned with 1 column volume (CV) of washing buffer (20 mM Tris pH 8.0, 120 mM NaCl) and the protein was eluted with high-imidazole buffer (20 mM Tris pH 8.0, 120 mM NaCl, 300 mM imidazole) until no enzyme was visible via the Bradford assay (~3.0 mL). Excess imidazole was subsequently removed using size exclusion chromatography (SEC) using PD-10 desalting columns packed with Sephadex G-25 resin (GE Healthcare) using 3.5 mL storage buffer (20 mM Tris pH 8.0, 120 mM NaCl). Protein purity (size: ~19.8 to 20.3 kDa) was assessed by SDS-PAGE using a 5-20% gradient gel. Protein concentrations were determined spectrophotometrically using the extinction coefficient (ε) in the range of 15930-24410 cm⁻¹ M⁻¹, as calculated with the ProtParam tool (https://web.expasy.org/protparam/). Additionally, concentrations were measured spectroscopically via the Bradford assay by adding the Coomassie reagent and recording the UV absorbance at 595 nm with a Thermo Scientific^{™} Varioskan^{™} LUX plate reader, using the bovine serum albumin (BSA) as the external standard.

### 4. Circular dichroism (CD) spectroscopy

The secondary structure of all lipase A variants was analyzed using CD spectroscopy. Protein samples were prepared at 5 µM concentration in a 1:1 mixture of storage buffer (20 mM Tris pH 8.0 and 120 mM NaCl) and PBS (10 mM, pH 7.4) in a 2 mm path length quartz cuvette. UV-CD spectra were recorded on a JASCO J-815 spectropolarimeter equipped with a Peltier temperature control unit at 25 °C. Data were collected from 200 to 300 nm with a 0.2 nm step size, 50 nm/min scanning speed, and a 2 nm bandwidth. CD spectra were acquired first at 25 °C using freshly prepared proteins, and then after heating up to 90 °C and cooling back to 25 °C to evaluate the *unfolding*/*folding* reversibility.

### 5. Thermal unfolding

Thermal unfolding was measured by monitoring the change in ellipticity at 222 nm as a function of temperature using a JASCO J-815 spectropolarimeter equipped with a Peltier temperature control unit. Protein samples were prepared at 5 µM in a 1:1 mixture of storage buffer (20 mM Tris pH 8.0 and 120 mM NaCl) and PBS (10 mM, pH 7.4) in a 2 mm quartz cuvette. The CD signal was recorded at a fixed wavelength (222 nm) while the temperature increased from 25 °C to 90 °C (*heating*) and then decreased from 90 °C to 25 °C (*cooling*) at the rate of 1 °C/min.

For each variant, the *heating* and *cooling* ellipticity values (θ) were rescaled to a [0,1] and the Tₘ values were determined by fitting normalized ellipticity values vs. temperature to a two-state (*folded*/*unfolded*) model as previously described (Núñez-Franco, R. et al., bioRxiv 2023, 556816).

### 6. Activity measurements

The kinetic parameters of mutants were determined spectrophotometrically at 25 °C using para-nitrophenylbutyrate (Bu-pNP) as a substrate using a 96-well microplate reader with temperature control. Different concentrated stock solutions ranging between 2-20 mM of Bu-pNP were prepared in acetonitrile in order to add a unique and well-defined volume to each reaction well and keep the amount of acetonitrile within the reaction well fixed at 5%. Briefly, 20 µL of the enzyme stock solution (1 mM in 20 mM Tris buffer pH 8.0, 120 mM NaCl) were added to 170 µL 20 mM Tris buffer pH 8.0, 120 mM NaCl and incubated at 25 °C for 5 min for equilibration. The reaction was started by adding 20 µL of the corresponding Bu-pNP stock solution, to yield controlled and varying concentrations in the range of 100 to 1000 µM, and the rate of increase in absorbance at 405 nm was recorded for 1 h. Spontaneous hydrolysis of Bu-pNP was monitored under identical conditions in the absence of enzyme. Absorption signal was converted to concentration using a calibration curve of the chromophore para-nitrophenol (pNP-OH). Reactions were performed in triplicate and each replicate was separately fitted to a Lineweaver-Burk plot to derive the values for K_{M}, k_{cat} and the catalytic efficiency (k_{cat}/K_{M}).

### 7. Aggregation of lipase A variants

Wild-type lipase A (WT) and the engineered variants were analyzed by size exclusion high-performance liquid chromatography (SEC-HPLC) using a system (Shimadzu, Tokyo, Japan) equipped with a photodiode array detector (254 nm). The mobile phase was composed of 50 mM phosphate, 300 mM NaCl buffer (pH 6.8) and the flow rate was set to 0.35 mL/min. 10 µL of the enzyme stock solutions (1 mM in 20 mM Tris buffer pH 8.0, 120 mM NaCl) were injected into a Biozen dSEC-2 column (3 µm, 200 Å, 300 mm x 4.6 mm, Phenomenex).

### 8. Thermal inactivation of lipase A variants

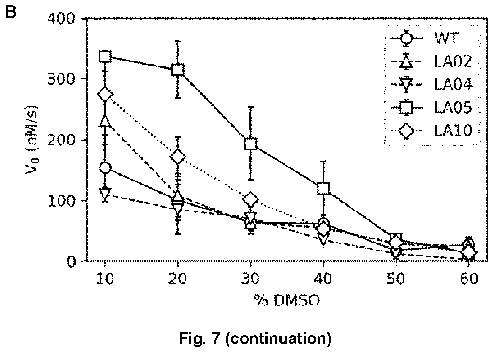

To determine resistance of wild-type lipase A and the designed variants to thermal inactivation, enzymes were pre-incubated at different temperatures for 20 min in Tris buffer (20 mM, 120 mM NaCl, pH 8.0) for 20 min. Then, they were cooled down to 4 °C in an ice bath (20 min) and subsequently, warmed up to the reaction temperature (25 °C) for 15 minutes. Samples were centrifuged to remove any possible aggregated/precipitated protein. Activities were measured using a 96-well microplate reader with temperature control by reacting the enzymes (100 nM) with Bu-pNP (250 µM) in Tris buffer (20 mM, 120 mM NaCl, pH 8.0) and 5% acetonitrile at 25 °C, recording the increase in absorbance at 405 nm for 20 min. The absorption signal was converted to concentration using a calibration curve of the chromophore pNP-OH. Reactions were performed in triplicate and the initial velocities were determined from the first 10 points (200 s). The residual activity (Figure 7) was measured as the relative conversion after 20 min reaction of the enzyme where 100% is the conversion after 20 min reaction upon pre-incubation of the corresponding enzyme at 25 °C.

### 9. Tolerance of lipase A variants to dimethyl sulfoxide

The tolerance of wild-type lipase A and the designed variants to the presence of organic solvents such as dimethyl sulfoxide (DMSO), was determined spectrophotometrically at 25 °C using *para*-nitrophenyloctanoate (Oct-pNP) as a substrate using a 96-well microplate reader with temperature control. A 2 mM stock solution of Oct-pNP was prepared in DMSO. Briefly, 4 µL of enzyme stock solution (1 mM in 20 mM Tris buffer pH 8.0, 120 mM NaCl) were added to 170 µL of 20 mM Tris buffer pH 8.0, 120 mM NaCl containing different amounts of DMSO and incubated at 25 °C for 5 min for equilibration. The reaction was started by adding 20 µL of the Oct-pNP stock solution, getting varying quantities of DMSO in the range of 10-60%, and the rate of increase in absorbance at 405 nm was recorded for 1 h. Final concentrations: 20 nM enzyme, 100 µM Oct-pNP. Absorption signal was converted to concentration using a calibration curve of pNP-OH. Reactions were performed in triplicate and the initial velocities were determined from the first 5 points (100 s).

### 10. Molecular dynamics simulations

Molecular dynamics simulations were performed on the wild-type lipase A and the designed variants LA04, LA05 and LA10 in complex with Bu-pNP as the substrate in 1) the unbound state, 2) the non-covalent bound state, 3) the covalent bound state forming the tetrahedral intermediate, 4) the covalent bound state forming the ester intermediate. These simulations were carried out with the *pmemd.cuda* module of AMBER 22 (Case, D. A., et al., Amber 2022; University of California, San Francisco, 2022) package implemented with the ff19SB (Tian, C., et al., J. Chem. Theory Comput. 2020, 16, 528-552) and GAFF2 (general Amber force field) force fields (Wang, J. et al., J. Comput. Chem. 2004, 25, 1157-1174). Parameters for the ligand and modified serine residue upon reaction with the ligand were generated with the antechamber module of AMBER, using the GAFF2 force field and with partial charges determined by fitting the electrostatic potential generated from a quantum mechanics calculation at the HF/6-31G(d) level of theory using the RESP method (Bayly, et al., J. Phys. Chem. 1993, 97, 10269-10280). Charges were calculated according to the Merz-Singh-Kollman scheme using Gaussian 16. The starting coordinates for the enzymes in the unbound state were generated by generating a model with AlphaFold (*monomer* parameters, *model_3* parameters).

Protein complexes were immersed in a water box with a 10 Å buffer of OPC3 (Izadi, S. et al., J. Phys. Chem. Lett. 2014, 5, 3863-3871) water molecules and neutralized by adding explicit Na⁺ or Cl⁻ counterions (Li-Merz 12-6 normal usage set) (Sengupta, A.; et al., J. Chem. Inf. Model. 2021, 61, 869-880). A two-stage geometry optimization approach was implemented. The first stage minimizes only the positions of solvent molecules and ions, and the second stage is an unrestrained minimization of all the atoms in the simulation cell. The systems were then heated by incrementing the temperature from 0 to 300 K under a constant pressure of 1 atm and periodic boundary conditions. Harmonic restraints of 10 kcal mol⁻¹ were applied to the solute, and the Andersen temperature coupling scheme (Andersen, H. C., J. Chem. Phys. 1980, 72, 2384-2393) was used to control and equalize the temperature. The time step was kept at 1 fs during the heating stages, allowing potential inhomogeneities to self-adjust. Water molecules were treated with the SHAKE algorithm (Miyamoto, S., et al., J. Comput. Chem. 1992, 13, 952-962) such that the angle between the hydrogen atoms is kept fixed through the simulations. Long-range electrostatic effects were modelled using the particle mesh Ewald method (Darden, T.; et al., J. Chem. Phys. 1993, 98, 10089-10092). An 8 Å cutoff was applied to Lennard-Jones interactions. Each system was equilibrated for 2 ns with a 2 fs time step at a constant volume and temperature of 300 K; production was run as 5 independent trajectories for an additional 200 ns under the same simulation conditions. The analysis of the trajectories was performed with *cpptraj* (Roe, D. R.; Cheatham, T. E., J. Chem. Theory Comput. 2013, 9, 3084-3095).

### Results

### First round of lipase A sequence design

lipase A was engineered following the general strategy of sampling positions characterized by low conservation using ProteinMPNN. In the current work, low conservation positions were identified computing the Shannon Entropy (Shannon, C. E., Bell Syst. Tech. J. 1948, 27, 379-423) on multiple sequence alignments of proteins sharing a high sequence identity (≥70%) with wild-type lipase A and then sampled with ProteinMPNN. Analysis of the sequences generated by ProteinMPNN allowed identifying recurrent mutations effectively increasing the thermal stability and activity of lipase A.

The first round of design started with wild-type lipase A and yielded two engineered variants, VarE and VarF. These variants are mutated at positions 49, 144, 161 and 167. They contain the mutations Y49E, D144E, D144S, and Y161T, and S167D (Table 3). A subsequent analysis yielded four more variants (LA01-LA04, Table 3) comprising the mutations of VarE and VarF The thermostability of these variants was assessed by monitoring the temperature-induced unfolding using circular dichroism, which showed that all the designed variants had higher Tₘ values than the wild-type (ΔTₘ, +4-11 °C, Figure 1A). This increase in Tₘ values indicates a substantial improvement in the thermostability of the designed variants, making them more robust under high-temperature conditions.

**Table 3. Number of mutations and mutation identities of the four engineered lipase A variants obtained in the first round of design. Reference is the wild-type sequence.**

| **Variant** | **Number of mutations** | **Mutations** | **SEQ ID NO** |
|---|---|---|---|
| varE | 4 | Y49E, D144E, Y161T, S167D | 2 |
| varF | 4 | Y49E, D144S, Y161T, S167D | 3 |
| LA01 | 7 | W42P, Y49E, M134T, M137P, D144E, Y161T, S167D | 4 |
| LA02 | 6 | Y49E, M134T, M137P, D144E, Y161T, S167D | 5 |
| LA03 | 9 | A20D, S24D, W42P, Y49E, R57A, K112T, D144E, Y161T, S167D | 6 |
| LA04 | 11 | A20D, S24D, W42P, Y49E, R57A, K112T, M134T, M137P, D144E, Y161T, S167D | 10 |
| LA05 | 10 | A20D, S24D, W42P, Y49E, R57A, K112T, M137P, D144E, Y161T, S167D | 8 |
| LA06 | 10 | A20D, S24D, Y49E, R57A, K112T, M134T, M137P, D144E, Y161T, S167D | 9 |
| LA07 | 9 | A20D, S24D, Y49E, R57A, K112T, M137P, D144E, Y161T, S167D | 7 |
| LA08 | 40 | I12V, S16A, F17S, A20D, G21A, S24A, Q29K, S32D, R33A, D34S, W42P, T47A, Y49E, N50K, R57A, Q60D, D64K, K69D, N94D, R107A, G111T, K112T, L114D, S131T, M134T, M137P, Y139S, R142W, D144S, R147K, Q150L, H152S, G155D, I157Y, Y161T, Q164E, N166F, S167D, I169V, N174D | 14 |
| LA09 | 29 | I12V, S16A, F17S, A20D, G21A, S24D, S32D, W42P, Y49E, N50K, R57A, Q60D, N94D, K112T, L114D, M134T, M137P, Y139S, D144E, R147K, Q150L, H152S, G155D, Y161T, Q164E, N166F, S167D, I169V, N174D | 13 |
| LA10 | 20 | S16A, F17S, A20D, S24D, W42P, Y49E, R57A, Q60D, N94D, K112T, M134T, M137P, D144E, R147K, H152S, Y161T, N166F, S167D, I169V, N174D | 12 |
| LA11 | 24 | F17S, A20D, G21A, S24D, S32D, D34S, W42P, Y49E, R57A, Q60D, D64K, N94D, K112T, M134T, M137P, Y139S, R142Y, D144E, R147K, Q150L, H152S, Y161T, N166F, S167D | 15 |
| LA12 | 17 | F17S, A20D, S24D, W42P, Y49E, R57A, Q60D, N94D, K112T, M134T, M137P, D144E, R147K, H152S, Y161T, N166F, S167D | 11 |
| LAL201 | 28 | E2S, A15S, F17D, A20D, G21N, S32D, R33E, W42S, T47S, Y49E, N50K, V54E, S56E, R57K, D64K, K69E, N94D, A97R, G111S, K112E, Y139E, R142E, D144K, H152S, Y161N, Q164E, S167D, Q178E | 24 |
| LAL202 | 16 | E2S, A20D, S32D, R33E, W42S, T47S, Y49E, N50K, V54E, S56E, R57K, K69E, N94D, H152S, Q164E, S167D | 22 |
| LAL203 | 12 | A20D, G21N, S24D, Y49E, N50K, V54Q, S56E, R57K, D64K, Y139E, Q164E, S167E | 21 |
| LAL204 | 8 | G21N, S24D, Y49E, N50K, V54Q, R57K, Y139E, Q164E | 19 |
| LAL205 | 20 | A20D, G21Y, S24D, Q29K, N48Q, Y49E, N50K, V54Q, S56E, R57K, Q60N, K61S, D64K, Y139E, S163E, Q164E, N166A, S167E, L168Y, K170Y | 23 |
| LAL206 | 10 | A20D, G21Y, S24D, Q29K, Y49E, N50K, R57K, Q60N, D64K, Q164E | 20 |

Notably, whereas wild-type lipase A has a completely irreversible temperature-induced unfolding, all the designed variants in this first round show some degree of unfolding reversibility upon heat treatment (15-50%, Figure 2, Table 4). The partial unfolding reversibility is particularly advantageous, as it suggests that these variants can regain their functional conformation after heat stress, potentially leading to longer enzyme lifetimes and greater efficiency.

**Table 4. Experimentally measured unfolding (T_{m,u}) and (re)folding (T_{m,f}) temperature, differences in unfolding Tₘ (ΔTₘ) and reversibility.**

| **Variant** | **T_{m,u} (°C)*^{a}*** | **T_{m,f} (°C)*^{b}*** | **Reversibility (%)** | **ΔTₘ (°C)*^{a,c}*** | **ΔTₘ (°C)*^{a,d}*** |
|---|---|---|---|---|---|
| WT | 54.0 | *n.d.* | -3.7*^{e}* | 0.0 | |
| LA01 | 61.8 | 60.8 | 30.6 | +7.8 | |
| LA02 | 62.8 | 61.5 | 18.4 | +8.8 | |
| LA03 | 57.7 | 51.0 | 13.9 | +3.7 | |
| LA04 | 64.6 | 61.9 | 50.4 | +10.6 | 0.0 |
| LA05 | 63.9 | 61.4 | 72.1 | +9.9 | -0.7 |
| LA06 | 64.4 | 62.5 | 64.0 | +10.4 | -0.2 |
| LA07 | 63.5 | 61.1 | 61.0 | +9.5 | -1.1 |
| LA08 | 72.9 | 69.7 | 87.6 | +18.9 | +8.3 |
| LA09 | 66.6 | 64.6 | 58.3 | +12.6 | +2.0 |
| LA10 | 63.5 | 61.9 | 43.7 | +9.5 | -1.1 |
| LA11 | 73.9 | 70.3 | 47.9 | +19.9 | +9.3 |
| LA12 | 65.5 | 63.0 | 10.7 | +11.5 | +0.9 |

| | | | | | |
|---|---|---|---|---|---|
| *^{a}Measured during forward thermal unfolding (u) (i.e. heating). ^{b}Measured during reverse thermal folding (f) (i.e. cooling). ^{c}Calculated with respect to wild-type lipase A. ^{d}Calculated with respect to LA04. ^{e}Negative reversibility values are due to numerical errors in the fitting of ellipticity values. In such* cases, *the unfolding is assumed to be totally irreversible (i.e. reversibility = 0%). n.d. Not determined due to irreversibility.* | | | | | |

Among the four variants, LA04 showed the best increase in Tₘ and reversibility, with an 11 °C enhancement in Tₘ and 50% in reversibility. LA04 contains 11 mutations (Figure 1B), all located at the protein surface. Two of them (M134T and M137P) are close to the catalytic triad. The mutations include two from positively charged to neutral (R57A and K112T) and four from neutral to negatively charged amino acids (A20D, S24D, Y49E, and S167D), as well as two mutations to proline (W42P and M137P).

Next, the catalytic activities of the wild-type lipase A and the variants LA01-04 were analyzed using 100 nM of each enzyme and increasing amounts (100-1000 µM) of chromogenic para-nitrophenylbutyrate (Bu-pNP) as a substrate. The concentration of the hydrolyzed para-nitrophenol (pNP-OH) product was measured over time by recording the absorbance at 405 nm (Figures 3A). All the variants were active as they showed higher conversion rates than the control reaction in the absence of enzymes. LA02 and LA04 displayed faster time-course profiles compared to the wild-type, whereas LA01 and LA03 showed significantly slower profiles. Examining the overall catalytic efficiency (k_{cat}/K_{M}, Figure 3B and Table 5), an increase was observed for variants LA02 and LA04, whereas it decreased for variants LA01 and LA03, in agreement with the time-course profiles. Remarkably, although not specifically designed for enhanced activity, variants LA02 and especially LA04 showed superior catalytic performance compared to the wild-type. This unexpected improvement highlights the potential of these variants for applications requiring higher enzymatic efficiency. These results underscore the delicate and intricate balance involved in enzyme design, where even small changes in sequence can have significant impacts on activity. In this sense, LA01, which has an additional mutation (W42P) compared to LA02, exhibits worse activity, suggesting that this extra mutation is detrimental to the enzyme's function. Conversely, LA03 and LA04 differ by only two additional mutations (M134T, M137P), which appear to significantly enhance activity. These observations highlight the complexity of predicting the effects of specific mutations based on a sole parameter (i.e. unfolding temperature, Tₘ) and the importance of iterative testing and optimization in enzyme engineering.

**Table 5. Kinetic parameters of wild-type lipase A (WT) and the designed variants (LA01-LA12) for Bu-pNP hydrolysis in 20 mM Tris buffer pH 8.0, 120 mM NaCl and 5% acetonitrile at 25 °C. The data are mean values ± standard deviation of triplicates experiments. N.**

| **Variant** | **k_{cat}/K_{M} (·10³ s⁻¹ M⁻¹)** | **k_{cat} (s⁻¹)** | **K_{M} (µM)** | **Relative k_{cat}/K_{M}** |
|---|---|---|---|---|
| WT | 6.42 ± 0.63 | 14.87 ± 10.78 | 2518.19 ± 2066.11 | 1.0 |
| LA01 | 2.37 ± 0.10 | 6.72 ± 0.26 | 2835.15 ± 27.30 | 0.4 |
| LA02 | 10.04 ± 0.29 | 12.45 ± 2.29 | 1245.07 ± 251.83 | 1.6 |
| LA03 | 1.20 ± 0.11 | 1.71 ± 0.30 | 1417.46 ± 196.28 | 0.2 |
| LA04 | 15.75 ± 1.72 | 6.50 ± 1.07 | 424.65 ± 109.07 | 2.5 |
| LA05 | 20.89 ± 0.49 | 19.66 ± 3.43 | 945.37 ± 187.39 | 3.3 |
| LA06 | 11.83 ± 3.33 | 6.62 ± 1.90 | 682.09 ± 437.76 | 1.8 |
| LA07 | 20.28 ± 0.56 | 18.59 ± 2.67 | 920.33 ± 153.10 | 3.2 |
| LA08 | 4.72 ± 1.41 | 0.48 ± 0.09 | 121.01 ± 68.32 | 0.7 |
| LA09 | 16.05 ± 3.82 | 1.99 ± 0.29 | 133.94 ± 44.55 | 2.5 |
| LA10 | 19.63 ± 2.51 | 12.39 ± 3.42 | 665.04 ± 273.24 | 3.1 |
| LA11 | 13.53 ± 2.00 | 3.49 ± 0.82 | 273.83 ± 107.29 | 2.1 |
| LA12 | 4.05 ± 2.38 | 1.29 ± 0.53 | 514.23 ± 390.11 | 0.6 |

### Second round of lipase A sequence design

Considering that LA04 showed the best properties in terms of both Tₘ and catalytic efficiency (i.e. k_{cat}/Kₘ), it was selected as the starting point for a second round of design. First, three variants, LA05-LA07 (Table 5), were rationally designed with the aim of determining the effect of specific mutations that induced significant differences in the properties of the variants designed in the first round. LA05 and LA06 recovered native residues M142 and W50, respectively, and LA07 recovered both mutations. On the other hand, the inventors decided to expand the pool of variable positions for this second round of design by lowering the Shannon Entropy threshold. This resulted in five additional variants (LA08-LA12, Table 6), with an average of 26 mutations over the wild-type (15 mutations over LA04). All of them could be expressed and purified following the same protocol showing a clean band pattern on an SDS-PAGE gel and an intense band around 20 kDa, indicating a high yield and purity. However, variants LA09 and LA12 showed a much weaker band, indicating lower expression yields.

**Table 6. Variable positions defined as set3 and set4.**

| *set3* | *set4* |
|---|---|
| 2 17 20 21 24 27 32 33 34 35 39 42 47 49 54 57 60 61 64 69 89 94 97 107 110 111 112 131 132 134 139 142 144 147 150 152 161 166 167 168 | 2 12 16 17 20 21 24 27 28 29 32 33 34 35 3639424749505457585960616465 69 89 94 96 97 102 107 109 110 111 112 114 128 131 132 134 137 139 142 144 147 149 150 151 152 154 155 157 161 162 164 166 167 168 169 174 178 |

As with the first-round variants, their Tₘ values were measured by monitoring temperature-induced unfolding using circular dichroism which, to our delight, showed similar or even higher values than LA04 (from -1 to +10 °C), as well as a higher unfolding reversibility reaching up to 88% for LA08 (Table 4). Among them, LA11 showed the highest Tₘ increase, with a gain of +20 °C with respect to wild-type lipase A and a 48% unfolding reversibility upon heat treatment. This variant contains 13 additional mutations over LA04 (24 over WT), all located at the protein surface and evenly distributed among loops and α-helices (Figure 4). Unlike the previous round, only a small net charge shift is observed (-1).

In terms of catalytic activity, all these variants, except LA08 and LA12, showed significant improvements in catalytic efficiency with respect to the wild-type, and some of showed an improvement over LA04 (Figure 3B and Table 5). Focusing on single mutants, LA05, which recovers M134 and maintains the mutation W42P, showed an increase in activity (1.4 and 3.3-fold with respect to LA04 and WT, respectively). This observation was unexpected, as mutation W42P was apparently detrimental for activity when comparing LA01 and LA02. Conversely, LA06, which recovers W42 and maintains mutation M134T showed a 0.6-fold decrease in activity with respect to LA04 (1.8-fold increase with respect to WT). Most strikingly, LA07, which recovered both mutations, has virtually the same activity as LA05, highlighting the synergy occurring between such mutations and how delicate and complicated it is to predict the effect of specific mutations, particularly in rounds of design/evolution in which many mutations appear all at once, a phenomenon known as sign epistasis.

Among the variants of the second round, LA10 showed the highest increase in activity. This variant contains 9 additional mutations compared to LA04 (20 compared to WT) located entirely at the protein surface and evenly spread across loops and α-helices and showed similar increments in Tₘ and unfolding reversibility upon heat treatment to LA04 (ΔTₘ = +9.5 °C, reversibility = 44%). On the other hand, LA08, which showed a significant increase in Tₘ (ΔTₘ = +19 °C) and almost complete reversibility upon heat treatment (88%), has 40 mutations over WT (31 over LA04). That vast number of mutations affect detrimentally catalysis, particularly mutations I12V, G155D and I157Y might contribute most considering they are near the catalytic triad.

The inventors set out to ascertain if the observed Tₘ values of the engineered lipase A variants could be predicted in silico as a further tool to reduce the number of sequences to screen experimentally in future perspective computational engineering campaigns. To this end, the mAFmin protocol developed in the inventor's group was used (Peccati, F., et al., J. Chem. Inf. Model. 2023, 63, 898-909), which combines AlphaFold structure prediction with scoring through the Rosetta (Leman, J., et al., Nat. Methods 2020, 17, 665-680) energy function. This approach has been previously validated on lipase A variants engineered through directed evolution and showed excellent correlation between calculated changes in folding free energy (ΔΔG_{f}) and changes in Tₘ values measured experimentally (Peccati, F., et al., J. Chem. Inf. Model. 2023, 63, 898-909). Such correlation for lipase A variants LA01-LA12 is shown in Figure 4 and is again very high (Pearson's coefficient = -0.96), indicating that lipase A from *Bacillus Subtilis* is an excellent system for in silico stability engineering and that mAFmin can be used to accurately predict thermal stability changes in designed variants.

### Molecular dynamics simulations

With the aim of studying the effects of substrate on the structure and the dynamics of the designed enzymes and shedding some light on the rationale for the observed enhanced properties, molecular dynamics (MD) simulations were performed. First, the enzymes in their apo state, i.e. in the absence of ligand, were studied to analyze their dynamics in solution. In general, the three analyzed variants with enhanced properties (LA04, LA05 and LA10) showed similar dynamics to wild-type lipase A. By analyzing the per-residue atomic fluctuations and comparing them with those of the wild-type lipase A, we did not identify any specific region with lower flexibility than wild-type. However, all designed variants are slightly more flexible in the loop where the mutation W42P is located. This is somewhat counter-intuitive, since the mutations to proline usually increase the rigidity of that area and, in theory, the stability of the protein. Similarly, the loop-helical region comprised between residues 12-23 also shows larger fluctuations than the wild-type, particularly for variants LA04 and LA05, which contain the A20D mutations. Conversely, variant LA10, which has two additional mutations in the same segment, S16A and F17S, exhibit similar fluctuations to the wild-type lipase A.

We then performed MD simulations for the non-covalent Bu-pNP/lipase complexes, simulating the pre-catalytic step of the reaction in which the substrate is bound at the active site. Unfortunately, these simulations did not provide any useful information, because although the ligand was initially bound to the active site, it partially left the binding site shortly after the beginning of the simulation, and in some of the simulations it even separated completely, going into the bulk solution. These weak and transient interactions between the substrate and the enzymes are consistent with the relatively high Kₘ values (high micromolar - low millimolar range) observed experimentally (Table 5).

MD simulations were also performed for the tetrahedral intermediates with Bu-pNP covalently bound to the enzyme, following the nucleophilic attack of the catalytic Ser77 on the carbonyl group of the ester. In this case, the ligand remained in the active site with virtually identical spatial arrangement in all simulations, irrespective of the enzyme, and thus provided no relevant information.

Finally, MD simulations were performed for the covalently bound serine butyrate intermediates upon release of the pNP-OH moiety (the acyl enzyme complex). Interestingly, the overall dynamics observed for the ligand-bound simulations were remarkably similar to those in the unbound state: the designed variants LA04, LA05, and LA10 continued to display comparable global flexibility to the wild-type lipase A, with no significant stabilization effects induced by the presence of the substrate; the loop region containing the W42P mutation remained more flexible compared to the wild-type enzyme; and the loop-helical region spanning residues 12-23 also exhibited enhanced fluctuations in LA04 and LA05, while LA10 maintained dynamics similar to the wild-type. On the other hand, the average distance between the catalytic His156 and the modified Ser77 residue was significantly larger throughout the simulations in the designed variants compared to the wild-type lipase A. Such a shift might reduce the likelihood of unfavorable interactions or strain during the catalytic cycle, which in turn might contribute to the enhanced activity of the designed variants.

### Aggregation and unfolding kinetics of lipase A variants

Wild-type lipase A has previously been described as a protein with a tendency to irreversible aggregation, especially at high concentrations, high temperatures and high pH values. However, aggregation has also been observed at room temperature and at pH above 7, and even inside chromatographic columns (Augustyniak, W.; et al., Protein Sci. 2012, 21, 487-497). Indeed, during a dialysis process in which wild-type lipase A was incubated overnight at 4 °C in a bath containing Tris buffer (20 mM Tris pH 8.0, 120 mM NaCl), we observed remarkable precipitation, turning the initially clear and translucent solution into opaque and cloudy. The designed variants do not show this behavior, indicating a lower aggregation propensity, probably due to the occurrence of mutations to charged amino acids on the protein surface favoring solubility and minimizing ionic interactions between the monomers. Then the aggregation propensity of wild-type lipase A and the engineered variants was analyzed by size-exclusion high-performance liquid chromatography (SEC-HPLC), which allows monitoring protein aggregation by separating molecules based on their hydrodynamic size (Schrag, D.; et al., Methods Mol. Biol. 2014, 1131, 507-512). In general, all the enzymes analyzed, including the wild-type lipase A, show similar chromatograms (Figure 5), with one major peak, one or more minor peaks, and in some cases overlapping (i.e., shoulder) peaks, suggesting the presence of oligomeric species. Some of them (LA01, LA03, LA05, and LA06) showed a dominant sharp peak, indicating minimal aggregation under the used experimental conditions. However, other variants (LA04, LA08, LA09 and LA11) show a higher number of peaks, indicating heterogeneity in the sample, possibly due to the presence of low-weight oligomers or aggregation intermediates. However, this does not seem to be directly related either to the activity of the enzymes or to their Tₘ values, nor to the aforementioned observation that precipitates appear in the wild-type lipase A after overnight incubation at 4 °C, which may indicate certain reversibility of the aggregation process.

Additionally, the unfolding kinetics of wild-type lipase A and a few selected variants with enhanced properties (i.e., LA04, LA05, LA11) was evaluated (Figure 6). To this end, enzymes were incubated at 25 °C and CD spectra were recorded at different times, showing that the wild-type rapidly loses its secondary structure (50% in 2 to 3 h), while the designed variants retain their structures for significantly longer times (>50% in 60 h). This correlates with the fact that the wild-type precipitates upon overnight incubation at 4 °C and indicates that the designed enzymes are substantially more thermostable and therefore more durable under the reaction conditions.

### Activity of lipase A variants under thermal stress

The thermostability of the wild-type lipase A and the designed variants was further investigated by determining their T₅₀ values, defined as the temperature at which 50% of the enzymatic activity is retained after a 20-minute incubation. Therefore, five lipase A variants with enhanced properties were selected (LA02, LA04, LA05, LA10, and LA11) and compared with the wild-type lipase A. All variants exhibited significant improvements in thermostability, with T₅₀ values above 60 °C, markedly higher than the T₅₀ value slightly below 40 °C observed for the wild-type. Variants from the first round of design showed T₅₀ values around 70 °C, while those from the second round showed values above 80 °C. In fact, LA05 and LA10 barely lose activity upon incubation at elevated temperatures. This feature is related to not only their increase in Tₘ, but also their ability to reverse the unfolding process upon heating and cooling cycles.

Finally, the performance of the wild-type lipase A and the designed variants in increasing amounts of dimethyl sulfoxide (DMSO) was assessed, since some of the reagents, particularly those bearing long aliphatic chains, are poorly soluble in aqueous media and may require high quantities of organic co-solvents. For example, *para-*nitrophenyloctanoate (Oct-pNP) is not completely soluble in Tris buffer (20 mM Tris pH 8.0, 120 mM NaCl) containing 5% DMSO, forming a grayish cloudy suspension. However, the solution becomes clearer at higher DMSO concentrations, being completely clear above 40% DMSO. Thus, four selected lipase A variants with enhanced properties (LA02, LA04, LA05, and LA10), as well as the wild-type lipase A, were used as catalysts for Oct-pNP hydrolysis in the presence of increasing amounts of DMSO. Although reactivity decreases as the DMSO concentration increases in all cases, the designed lipase A variants, particularly LA05, have much higher activity than WT under the same conditions. Indeed, LA05 at 40% DMSO has a similar activity to the wild-type at 10% DMSO. Notably, variant LA04, which has shown significantly better catalytic properties than the wild-type when catalyzing the hydrolysis of Bu-pNP, shows slightly lower activities when reacting with Oct-pNP. However, LA05, which lacks the M142T mutation compared to LA04, shows much higher activities. This suggests that this residue, located very close to the active site, may participate in hydrophobic interactions with the aliphatic chain of the ligand. Hence, having a more polar Thr (LA04) instead of the native Met (LA05) is detrimental for activity towards more hydrophobic substrates.

## Claims

1. A lipase A enzyme variant having at least 80% identity with an amino acid sequence according to SEQ ID NO: 1, wherein the enzyme variant is **characterized by** comprising the mutations Y49E, D144E/S, Y161T, and S167D, and wherein the enzyme variant has improved thermostability and/or catalytic efficiency in comparison with the enzyme according to SEQ ID NO: 1.

2. The lipase A enzyme variant according to claim 1 further comprising one of the following mutations sets:
a) W42P, M134T, M137P, and D144E, wherein the variant comprises the sequence according to SEQ ID NO: 4;
b) M134T, M137P, and D144E, wherein the variant comprises the sequence according to SEQ ID NO: 5;
c) A20D, S24D, W42P, R57A, K112T, and D144E, wherein the variant comprises the sequence according to SEQ ID NO: 6; or
d) A20D, S24D, R57A, K112T, M137P, and D144E, wherein the variant comprises the sequence according to SEQ ID NO: 7.

3. The lipase A enzyme variant according to claim 2 wherein:
- the variant comprising the mutations A20D, S24D, W42P, R57A, K112T, and D144E, further comprises the mutation M137P, and wherein the variant comprises the sequence according to SEQ ID NO: 8;
- the variant comprising the mutations A20D, S24D, R57A, K112T, M137P, and D144E, further comprises the mutation M134T, and wherein the variant comprises the sequence according to SEQ ID NO: 9;
- the variant comprising the mutations W42P, K112T, M134T, M137P, and D144E, further comprises the mutations A20D, S24D, and R57A, and wherein the variant comprises the sequence according to SEQ ID NO: 10;
- the variant comprising the mutations W42P, K112T, M134T, M137P, D144E, A20D, S24D, and R57A further comprises the mutations F17S, Q60D, N94D, R147K, H152S and N166F, and wherein the variant comprises the sequence according to SEQ ID NO: 11;
- the variant comprising the mutations W42P, K112T, M134T, M137P, D144E, A20D, S24D, R57A, F17S, Q60D, N94D, R147K, H152S and N166F further comprises the mutations S16A, I169V, and N174D, and wherein the variant comprises the sequence according to SEQ ID NO: 12;
- the variant comprising the mutations W42P, K112T, M134T, M137P, D144E, A20D, S24D, R57A, F17S, Q60D, N94D, R147K, H152S, N166F, S16A, I169V, and N174D further comprises the mutations I12V, G21A, S32D, N50K, L114D, Y139S, Q150L, G155D, and Q164E, and wherein the variant comprises the sequence according to SEQ ID NO: 13;
- the variant comprising the mutations W42P, K112T, M134T, M137P, D144E, A20D, S24D, R57A, F17S, Q60D, N94D, R147K, H152S, N166F, S16A, I169V, and N174D, I12V, G21A, S32D, N50K, L114D, Y139S, Q150L, G155D, and Q164E further comprises the mutations D24A, Q29K, R33A, D34S, T47A, D64K, K69D, R107A, G111T, S131T, R142W, E144S, and I157Y, and wherein the variant comprises the sequence according to SEQ ID NO: 14; or
- the variant comprising the mutations W42P, K112T, M134T, M137P, D144E, A20D, S24D, and R57A, F17S, Q60D, N94D, R147K, H152S and N166F further comprises the mutations G21A, S32D, D34S, D64K, Y139S, R142Y, and Q150L, and wherein the variant comprises the sequence according to SEQ ID NO: 15.

4. The lipase A enzyme variant according to any one of claims 1 to 3 further comprising a marker.

5. The lipase A enzyme variant according to claim 4 wherein the marker is a polyhistidine peptide.

6. The lipase A enzyme variant according to claim 4 or 5, further comprising a flexible linker peptide between the marker and the lipase A enzyme variant.

7. The lipase A enzyme variant according to any one of claims 1 to 6, wherein the improvement in thermostability is of at least 1 °C in the unfolding temperature of the enzyme variant.

8. The lipase A enzyme variant according to any one of claims 1 to 7, wherein the improvement in catalytic efficiency is of at least 1% or more.

9. The lipase A enzyme variant according to any of claims 1 to 8 immobilized to a support.

10. The lipase A enzyme variant according to any of claims 1 to 8, wherein the enzyme is lyophilized.

11. A polynucleotide encoding the lipase A enzyme variant according to any one of claims 1 to 8.

12. A vector comprising the polynucleotide according to claim 11.

13. A host cell comprising the polynucleotide according to claim 11 or the vector according to claim 12.

14. A method for obtaining the lipase A enzyme variant according to anyone of claims 1 to 10 comprising the steps of:
a) culturing a host cell according to claim 13 and
b) recovering the lipase A enzyme variant from the culture medium or from the cell.

15. Use of the lipase A enzyme variant according to any one of claims 1 to 10 as an industrial biocatalyst, in food processing, as a detergent additive, as a biosensor, in the production of a cosmetic, or in the chemical synthesis of compounds, wherein the compound is selected from a pharmaceutical compound, an agrochemical compound or a pesticide compound.
